(19) **Europäisches Patentamt European Patent Office Office européen des brevets**

(11) **EP 4 454 566 B1**

(12) **EUROPEAN PATENT SPECIFICATION**

(45) Date of publication and mention of the grant of the patent:
**05.08.2026   Bulletin 2026/32**

(21) Application number: **22910624.0**

(22) Date of filing: **02.11.2022**

(51) International Patent Classification (IPC):
*A61B 6/00* (2024.01)          *G06T 7/00* (2017.01)
*A61B 6/50* (2024.01)

(52) Cooperative Patent Classification (CPC):
**G06T 7/0012; A61B 6/502; A61B 6/5217;**
A61B 6/54; G06T 2207/10116; G06T 2207/20081;
G06T 2207/30068; G06T 2207/30096

(86) International application number:
**PCT/JP2022/041084**

(87) International publication number:
**WO 2023/119904 (29.06.2023 Gazette 2023/26)**

(54) **IMAGE PROCESSING DEVICE, IMAGE PROCESSING METHOD, AND IMAGE PROCESSING PROGRAM**

BILDVERARBEITUNGSVORRICHTUNG, BILDVERARBEITUNGSVERFAHREN UND BILDVERARBEITUNGSPROGRAMM

DISPOSITIF DE TRAITEMENT D'IMAGE, PROCÉDÉ DE TRAITEMENT D'IMAGE, ET PROGRAMME DE TRAITEMENT D'IMAGE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.12.2021   JP 2021207601**

(43) Date of publication of application:
**30.10.2024   Bulletin 2024/44**

(73) Proprietor: **FUJIFILM Corporation Tokyo 106-8620 (JP)**

(72) Inventor: **MACHII, Yusuke Ashigarakami-gun, Kanagawa 258-8538 (JP)**

(74) Representative: **Dehns Germany Partnerschaft mbB Theresienstraße 6-8 80333 München (DE)**

(56) References cited:
CN-A- 107 798 679          JP-A- 2002 109 512
JP-A- 2005 177 037          JP-A- 2016 022 143
JP-A- 2018 161 405          JP-A- H08 294 479
JP-A- H09 251 535           US-A- 5 857 030
US-A- 5 946 407

• HAO DU ET AL: "Multi-task Graph Convolutional Neural Network for Calcification Morphology and Distribution Analysis in Mammograms", ARXIV.ORG, CORNELL UNIVERSITY LIBRARY, 201 OLIN LIBRARY CORNELL UNIVERSITY ITHACA, NY 14853, 14 May 2021 (2021-05-14), XP081965656

**Description**

Technical Field

The present disclosure relates to an image processing apparatus, an image processing method, and a non-transitory storage medium storing an image processing program.

Related Art

Calcification is interpreted in a radiation image captured by irradiating a breast with radiation. Therefore, a technique for supporting the interpretation of the calcification is known. For example, JP2018-161405A discloses a technique for enabling an intuitive determination of a portion in which a mammary gland region and a small round calcification region overlap each other. JP2016-22143A discloses a technique for intuitively understanding a dense state of a small round calcification tissue.

Hao, Du, et al.: "Multi-task Graph Convolutional Neural Network for Calcification Morphology and Distribution Analysis in Mammograms", arxiv.org, Cornell University Library, 201 Olin Library Cornell University Ithaca, NY 14853, 14 May 2021 (2021-05-14) discloses a multi-task deep graph convolutional network (GCN) method for the automatic characterization of morphology and distribution of micro calcifications in mammograms.

SUMMARY OF INVENTION

Technical Problem

Meanwhile, in a case in which a diagnosis is made based on the calcification in a case of performing the interpretation related to the calcification, a type of a distribution state of the calcification may be used. However, in the techniques of the above-described documents, it may be difficult to determine the type of the distribution state of the calcification. Solution to Problem

The present disclosure has been made in view of the above circumstances, and provides an image processing apparatus, an image processing method, and a non-transitory storage medium storing an image processing program which can accurately determine a type of a distribution state of calcification of a breast.

A first aspect of the present disclosure relates to an image processing apparatus including at least one processor, in which the processor is configured to: detect calcification from a radiation image obtained by imaging a breast by irradiating the breast with radiation; generate a low resolution image from a calcification distribution image representing a detection result of the calcification by reducing a resolution of the calcification distribution image to be lower than a resolution of the radiation image; and determine a type of a distribution state of the calcification based on the low resolution image.

In an embodiment, the calcification distribution image is a grayscale image or a binary image.

In an embodiment, the calcification distribution image is a mask image in which a region other than a region of the calcification is masked.

In an embodiment, the processor is configured to enlarge an image of the calcification included in the calcification distribution image and generate the low resolution image from the calcification distribution image in which the image of the calcification is enlarged.

In an embodiment, the processor is configured to: perform filtering on the calcification distribution image based on a signal value of the calcification; and generate the low resolution image from the calcification distribution image subjected to the filtering.

In an embodiment, the processor is configured to: derive a mammary gland amount of the breast from the radiation image; and set a threshold value for the filtering based on the mammary gland amount.

In an embodiment, the processor is configured to detect at least one of a skin line or a nipple of the breast from the radiation image, and the calcification distribution image includes at least one of the detected skin line or nipple.

In an embodiment, the processor is configured to, in a case in which the calcification distribution image includes a plurality of distributions, determine the type of the distribution state for each distribution.

In an embodiment, the radiation image includes a left breast radiation image obtained by imaging a left breast of a subject and a right breast radiation image obtained by imaging a right breast of the subject, and the processor is configured to: detect calcification from each of the left breast radiation image and the right breast radiation image; generate a left breast low resolution image from a left breast calcification distribution image representing a detection result of the calcification of the left breast; generate a right breast low resolution image from a right breast calcification distribution image representing a detection result of the calcification of the right breast; and determine a type of a distribution state of the calcification based on the left breast low resolution image and the right breast low resolution image.

In an embodiment, the processor is configured to determine a type of a shape of the calcification from the low resolution image.

In an embodiment, the processor is configured to estimate a degree of malignancy of the calcification based on the type of the distribution state of the calcification and the type of the shape of the calcification.

**In** an embodiment, the processor is configured to estimate whether the calcification is benign or malignant from the low resolution image.

**In** an embodiment, the processor is configured to display a determination result of the type of the distribution state in a form of being superimposed on the radiation image.

**In** an embodiment, the processor is configured to detect the calcification from the radiation image by using a rule-based calcification detection model.

**In** an embodiment, the processor is configured to detect the calcification from the radiation image by using a learning-based calcification detection model.

In an embodiment, the radiation image is a two-dimensional image obtained by normal imaging of the breast of a subject, a plurality of tomographic images obtained from a series of a plurality of projection images obtained by tomosynthesis imaging of the breast, or a composite two-dimensional image obtained by combining at least a part of the series of the plurality of projection images or the plurality of tomographic images.

A second aspect of the present disclosure relates to an image processing method performed by a computer, the method including: detecting calcification from a radiation image obtained by imaging a breast by irradiating the breast with radiation; generating a low resolution image from a calcification distribution image representing a detection result of the calcification by reducing a resolution of the calcification distribution image to be lower than a resolution of the radiation image; and determining a type of a distribution state of the calcification based on the low resolution image.

A third aspect of the present disclosure relates to non-transitory storage medium storing a program that causes a computer to execute image processing , the image processing including: detecting calcification from a radiation image obtained by imaging a breast by irradiating the breast with radiation; generating a low resolution image from a calcification distribution image representing a detection result of the calcification by reducing a resolution of the calcification distribution image to be lower than a resolution of the radiation image; and determining a type of a distribution state of the calcification based on the low resolution image.

Effects of Invention

According to the present disclosure, the type of the distribution state of the calcification of the breast can be accurately determined.

BRIEF DESCRIPTION OF THE DRAWINGS

Fig. 1 is a configuration diagram schematically illustrating an example of an overall configuration of a radiography system according to an embodiment.

Fig. 2 is a diagram illustrating an example of tomosynthesis imaging.

Fig. 3 is a block diagram illustrating an example of a configuration of an image processing apparatus according to the embodiment.

Fig. 4 is a diagram illustrating a classification and a type of calcification.

Fig. 5 is a diagram illustrating an example of a calcification distribution determination model.

Fig. 6 is a diagram illustrating convolution processing.

Fig. 7 is a diagram illustrating an example of training the calcification distribution determination model through machine learning.

Fig. 8 is a schematic diagram illustrating an outline of a flow of determining a type of a distribution state of the calcification in the image processing apparatus according to the embodiment.

Fig. 9 is a functional block diagram illustrating an example of the configuration of the image processing apparatus according to the embodiment.

Fig. 10 is a flowchart illustrating an example of a flow of image processing via the image processing apparatus according to the embodiment.

Fig. 11A is a diagram illustrating an example of display of a determination result of the type of the distribution state of the calcification.

Fig. 11B is a diagram illustrating an example of display of the determination result of the type of the distribution state of the calcification.

Fig. 12 is a block diagram illustrating an example of a storage unit of an image processing apparatus according to Modification Example 1.

Fig. 13 is a diagram illustrating an example of training a calcification detection model according to Modification Example 1 through machine learning.

Fig. 14 is a schematic diagram illustrating an outline of a flow of determining a type of a distribution state of calcification in an image processing apparatus according to Modification Example 2.

Fig. 15 is a flowchart illustrating an example of a flow of image processing via the image processing apparatus according to Modification Example 2.

Fig. 16 is a schematic diagram illustrating an outline of a flow of determining a type of a distribution state of calcification in an image processing apparatus according to Modification Example 3.

Fig. 17 is a flowchart illustrating an example of a flow of image processing via the image processing apparatus according to Modification Example 3.

Fig. 18 is a schematic diagram illustrating an outline of another example of the flow of determining the type of the distribution state of the calcification in the image processing apparatus according to Modification Example 3.

Fig. 19 is a schematic diagram illustrating an outline of a flow of determining a type of a distribution state of calcification in an image processing apparatus according to Modification Example 4.

Fig. 20 is a schematic diagram illustrating an outline of a flow of determining a type of a distribution state of calcification in an image processing apparatus according to Modification Example 5.

Fig. 21 is a schematic diagram illustrating an outline of a flow of determining a type of a distribution state of calcification in an image processing apparatus according to Modification Example 6.

Fig. 22 is a schematic diagram illustrating an outline of an example of a flow of determining a type of a shape of calcification in an image processing apparatus according to Modification Example 7.

Fig. 23 is a diagram illustrating an example of training a calcification shape determination model through machine learning.

Fig. 24 is a flowchart illustrating an example of a flow of image processing via the image processing apparatus according to Modification Example 7.

Fig. 25 is a schematic diagram illustrating an outline of an example of a flow of estimation of whether calcification is benign or malignant in an image processing apparatus according to Modification Example 8.

Fig. 26 is a diagram illustrating an example of training a benign/malignant estimation model through machine learning.

Fig. 27 is a flowchart illustrating an example of a flow of image processing via the image processing apparatus according to Modification Example 8.

## DESCRIPTION OF EMBODIMENTS

Hereinafter, an embodiment of the present disclosure will be described in detail with reference to the drawings. It should be noted that the present disclosure is not limited by the present embodiment.

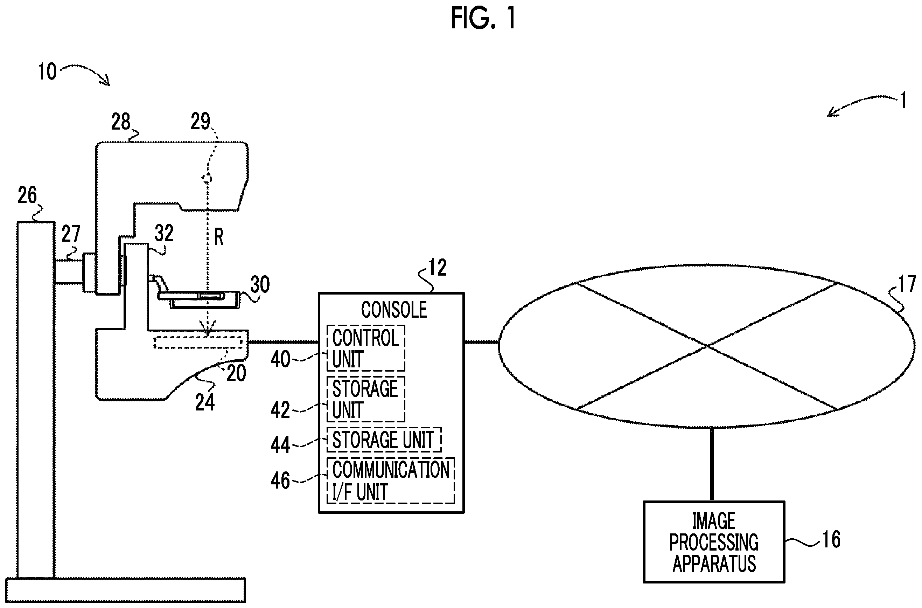

First, an example of an overall configuration of a radiography system according to the present embodiment will be described. Fig. 1 is a configuration diagram illustrating an example of the overall configuration of the radiography system 1 according to the present embodiment. As illustrated in Fig. 1, the radiography system 1 according to the present embodiment comprises a mammography apparatus 10, a console 12, and an image processing apparatus 16. The console 12 and the image processing apparatus 16 are connected to each other using wired communication or wireless communication via a network 17.

First, the mammography apparatus 10 according to the present embodiment will be described. Fig. 1 is a side view illustrating an example of an appearance of the mammography apparatus 10 according to the present embodiment. It should be noted that Fig. 1 illustrates an example of the appearance in a case in which the mammography apparatus 10 is viewed from a left side of a subject.

The mammography apparatus 10 according to the present embodiment is an apparatus that is operated in response to the control of the console 12, uses a breast of the subject as an object to irradiate the breast with radiation R (for example, X-rays) applied from a radiation source 29, and captures a radiation image of the breast. Further, the mammography apparatus 10 according to the present embodiment has a function of performing normal imaging that captures an image by arranging the radiation source 29 at an irradiation position along a normal direction to a detection surface 20A of a radiation detector 20 and so-called tomosynthesis imaging (which will be described in detail below) that captures images by moving the radiation source 29 to each of a plurality of irradiation positions.

As illustrated in Fig. 1, the mammography apparatus 10 comprises an imaging table 24, a base 26, an arm part 28, and a compression unit 32.

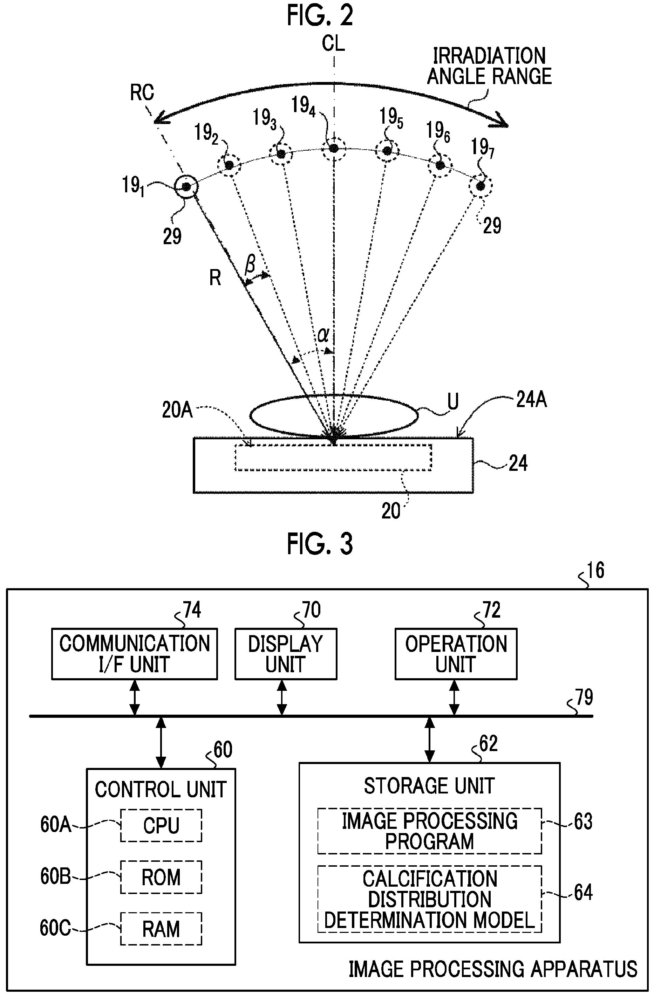

A radiation detector 20 is arranged inside the imaging table 24. As illustrated in Fig. 2, in the mammography apparatus 10 according to the present embodiment, in a case in which the imaging is performed, a breast U of the subject is positioned on an imaging surface 24A of the imaging table 24 by a user.

The radiation detector 20 detects the radiation R transmitted through the breast U as the object. Specifically, the radiation detector 20 detects the radiation R that enters the breast U of the subject and the imaging table 24 and reaches the detection surface 20A of the radiation detector 20, generates a radiation image based on the detected radiation R, and outputs image data representing the generated radiation image. Hereinafter, the series of operations of irradiating the breast with the radiation R from the radiation source 29 to generate the radiation image via the radiation detector 20 may be referred to as "imaging". A type of the radiation detector 20 according to the present embodiment is not particularly limited. For example, the radiation detector 20 may be an indirect conversion type radiation detector that converts the radiation R into light and converts the converted light into charge, or may be a direct conversion type radiation detector that directly converts the radiation R into charge.

A compression plate 30 used to compress the breast during the imaging is attached to the compression unit 32 provided on the imaging table 24 and is moved in a direction (hereinafter, referred to as "up-down direction") toward or away from the imaging table 24 by a compression plate driving unit (not illustrated) provided in the compression unit 32. The compression plate 30 is moved in the up-down direction to compress the breast of the subject between the imaging table 24 and the compression plate 30.

The arm part 28 can rotate with respect to the base 26 via a shaft part 27. The shaft part 27 is fixed to the base 26, and the shaft part 27 and the arm part 28 rotate as one body. Gears are provided in each of the shaft part 27 and the compression unit 32 of the imaging table 24, and by switching the gears between an engaged state and a non-engaged state, a state in which the compression unit 32 of the imaging table 24 and the shaft part 27 are connected to each other and rotate as one body and a state in which the shaft part 27 is separated from the imaging table 24 and idles can be switched. It should be noted that the elements for switching between transmission and non-transmission of power of the shaft part 27 are not limited to the gears, and various mechanical elements can be used. The arm part 28 and the imaging table 24 can rotate separately with respect to the base 26 with the shaft part 27 as a rotation axis.

In a case in which the tomosynthesis imaging is performed in the mammography apparatus 10, the radiation source 29 is sequentially moved to each of the plurality of irradiation positions having different irradiation angles in response to the rotation of the arm part 28. The radiation source 29 includes a radiation tube (not illustrated) that generates the radiation R, and the radiation tube is moved to each of the plurality of irradiation positions in response to the movement of the radiation source 29. Fig. 2 is a diagram illustrating an example of the tomosynthesis imaging. It should be noted that the compression plate 30 is not illustrated in Fig. 2. In the present embodiment, as illustrated in Fig. 2, the radiation source 29 is moved to the irradiation positions 19t (t = 1, 2, ..., the maximum value in the example of Fig. 2 is 7) at which the irradiation angles differ by a predetermined angle $\beta$, in other words, positions at which the irradiation angles of the radiation R with respect to the detection surface 20A of the radiation detector 20 differ. At each irradiation position 19t, the breast U is irradiated with the radiation R applied from the radiation source 29 in response to an instruction of the console 12, and the radiation image is captured by the radiation detector 20. In the radiography system 1, in a case in which the tomosynthesis imaging is performed by moving the radiation source 29 to each irradiation position $19_t$ to capture the radiation image at each irradiation position 19t, seven radiation images are obtained in the example of Fig. 2. It should be noted that, hereinafter, the radiation image captured at each irradiation position 19t in the tomosynthesis imaging is also referred to as "projection image" in a case of being distinguished from other radiation images. Further, in a case in which the radiation images, such as a projection image, a tomographic image which will be described below, and a normal two-dimensional image, are generically referred to regardless of the type, the radiation images are simply referred to as "radiation image".

It should be noted that, as illustrated in Fig. 2, the irradiation angle of the radiation R means an angle $\alpha$ formed by a normal line CL of the detection surface 20A of the radiation detector 20 and a radiation axis RC. The radiation axis RC means an axis that connects a focus of the radiation source 29 at each irradiation position 19t and a preset position, such as a center of the detection surface 20A. Here, it is assumed that the detection surface 20A of the radiation detector 20 is substantially parallel to the imaging surface 24A.

Moreover, in a case in which the mammography apparatus 10 performs the normal imaging, the radiation source 29 remains at the irradiation position $19_t$ (the irradiation position $19_t$ along the normal direction, the irradiation position $19_4$ in Fig. 2) at which the irradiation angle $\alpha$ is 0 degrees. The radiation R is applied from the radiation source 29 in response to the instruction of the console 12, and the radiation image is captured by the radiation detector 20. It should be noted that, in the present embodiment, in a case in which the radiation image obtained by the normal imaging is distinguished from the other radiation image such as the projection image, the radiation image is referred to as "normal two-dimensional image".

The mammography apparatus 10 and the console 12 are connected using wired communication or wireless communication. The radiation image captured by the radiation detector 20 in the mammography apparatus 10 is output to the console 12 using wired communication or wireless communication via a communication interface (I/F) unit (not illustrated).

As illustrated in Fig. 1, the console 12 according to the present embodiment comprises a control unit 40, a storage unit 42, a user I/F unit 44, and a communication I/F unit 46.

As described above, the control unit 40 of the console 12 has a function of controlling the capture of the radiation image of the breast via the mammography apparatus 10. Examples of the control unit 40 include a computer system comprising a central processing unit (CPU), a read only memory (ROM), and a random access memory (RAM).

The storage unit 42 has a function of storing, for example, information on the capture of the radiation image, or the radiation image acquired from the mammography apparatus 10. The storage unit 42 is a non-volatile storage unit and is, for example, a hard disk drive (HDD) or a solid state drive (SSD).

The user I/F unit 44 includes input devices, such as various buttons and switches operated by the user such as a technician, regarding the capture of the radiation image and display devices, such as a lamp and a display, that display information on the imaging or the radiation image.

The communication I/F unit 46 transmits and receives various types of data, such as information on the capture of the radiation image or the radiation image obtained via the imaging, to and from the mammography apparatus 10 using wired communication or wireless communication. In addition, the communication I/F unit 46 transmits and receives various types of data, such as the radiation image, to and from the image processing apparatus 16 via the network 17 using wired communication or wireless communication.

The image processing apparatus 16 is used by a doctor or the like (hereinafter, simply referred to as "doctor") to interpret the radiation image. The image processing apparatus 16 according to the present embodiment has a function of detecting faint calcification from the radiation image and displaying a calcification distribution image representing the faint calcification of the breast of the radiation image, but the detection and the display of the faint calcification are not essential.

Fig. 3 is a block diagram illustrating an example of a configuration of the image processing apparatus 16 according to the present embodiment. As illustrated in Fig. 3, the image processing apparatus 16 according to the present embodiment comprises a control unit 60, a storage unit 62, a display unit 70, an operation unit 72, and a communication I/F unit 74. The control unit 60, the storage unit 62, the display unit 70, the operation unit 72, and the communication I/F unit 74 are connected to each other via a bus 79, such as a system bus or a control bus, such that various types of information can be transmitted and received.

The control unit 60 controls the overall operation of the image processing apparatus 16. The control unit 60 comprises a CPU 60A, a ROM 60B, and a RAM 60C. Various programs and the like used by the CPU 60A for the control are stored in the ROM 60B in advance. The RAM 60C transitorily stores various types of data.

The storage unit 62 is a non-volatile storage unit and is, for example, an HDD or an SSD. Various types of information, such as an image processing program 63 and a calcification distribution determination model 64, are stored in the storage unit 62.

The calcification distribution determination model 64 is a model that outputs a determination result obtained by determining a type of a distribution state of the calcification in a case in which a low resolution image including the calcification is input.

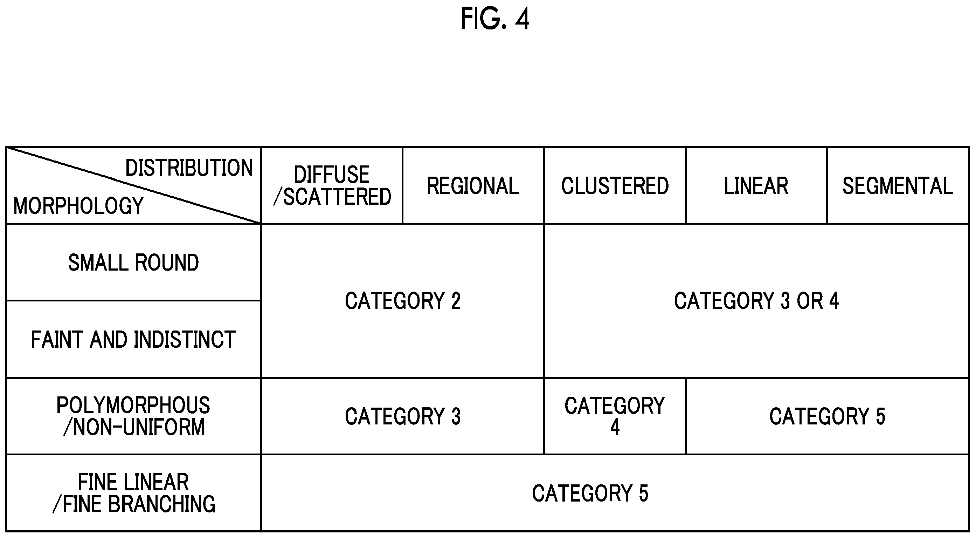

There are a plurality of types of calcification observed by, for example, the doctor. For example, according to a mammography guideline, the calcification observed from the radiation image is classified into typically benign calcification and calcification that requires discrimination between benignity and malignancy. Examples of the typically benign calcification include vascular calcification, lucent-centered calcification, milk-of-lime calcification, and suture calcification. Meanwhile, the calcification that should be requires discrimination between benignity and malignancy is calcification that does not belong to the typically benign calcification and is mainly classified depending on the calcification morphology and distribution as illustrated in Fig. 4. The calcification morphologies are classified into "small round", "faint and indistinct", "polymorphous or non-uniform", and "fine linear or fine branching". The "small round" calcification has a circular or elliptical shape of 1 mm or less, and is a clear marginal calcification. The "faint and indistinct" calcification is mainly a circular or flake-like calcification, and most of these types of calcification are small and faint. Since the calcification is faint and indistinct, it is difficult to classify the morphologies, and the calcification tends to be difficult to see in the radiation image. It should be noted that the faint and indistinct calcification tends to be smaller than the small round calcification. The "polymorphous or non-uniform" calcification is irregular calcification having various sizes and densities and typically has a broken stone shape. In addition, the "fine linear or fine branching" calcification is elongated irregular calcification and is generally recognized as being linear. Meanwhile, the calcification distributions are classified into "diffuse or scattered", "regional", "clustered", "linear", and "segmental". The "diffuse or scattered" means that the calcification is scattered in the entire breast without having a constant distribution tendency. The "regional" means that the calcification spreads over a wide area, but does not spread throughout the mammary gland. The "clustered" means that many pieces of calcification are confined to a small area. The "linear" means that the calcification is linearly arranged. The "segmental" means that the calcification matches a mammary ductal-lobular system and suggests that breast cancer is likely to spread to a glandular lobe or a segment.

In the mammography guideline, as illustrated in Fig. 4, categories are associated with the calcification distribution and morphology. "Category 2" indicates benign, "Category 3" indicates that the calcification is benign, but whose malignancy is not capable of being denied, "Category 4" indicates that the calcification is suspected to be malignant, and "Category 5" indicates malignant. In this way, the benign and malignant diagnosis of the calcification is performed depending on the calcification morphology and distribution.

The calcification distribution determination model 64 according to the present embodiment determines which of "diffuse or scattered", "regional", "clustered", "linear", or "segmental" is the distribution state of the calcification.

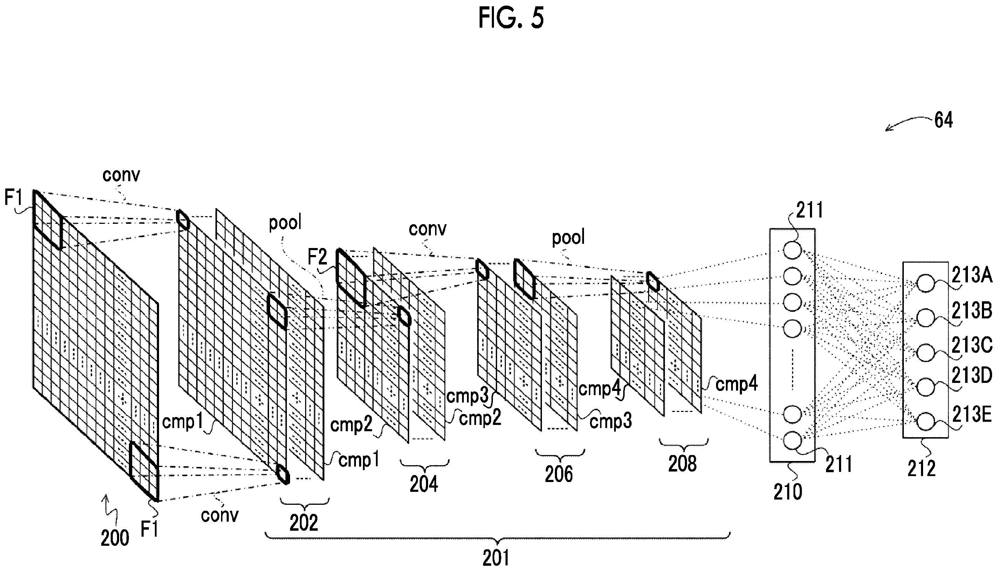

In the present embodiment, a convolutional neural network (CNN) that has been subjected to machine learning through deep learning using training data is used as the calcification distribution determination model 64. Fig. 5 illustrates an example of the calcification distribution determination model 64 according to the present embodiment.

The calcification distribution determination model 64 illustrated in Fig. 5 comprises an input layer 200, an intermediate layer 201, a flat layer 210, and an output layer 212. A processing target image (low resolution image described in detail below in the present embodiment) is input to the input layer 200. The input layer 200 propagates information for each pixel (per one pixel) on the input processing target image to the intermediate layer 201 as it is. For example, in a case in which the size of the processing target image is 28 pixels × 28 pixels and the data is a grayscale, the size of the data propagated from the input layer 200 to the intermediate layer 201 is 28 × 28 × 1 = 784.

The intermediate layer 201 includes a convolutional layer 202 and a convolutional layer 206 that perform convolution processing (conv), and a pooling layer 204 and a pooling layer 208 that perform pooling processing (pool).

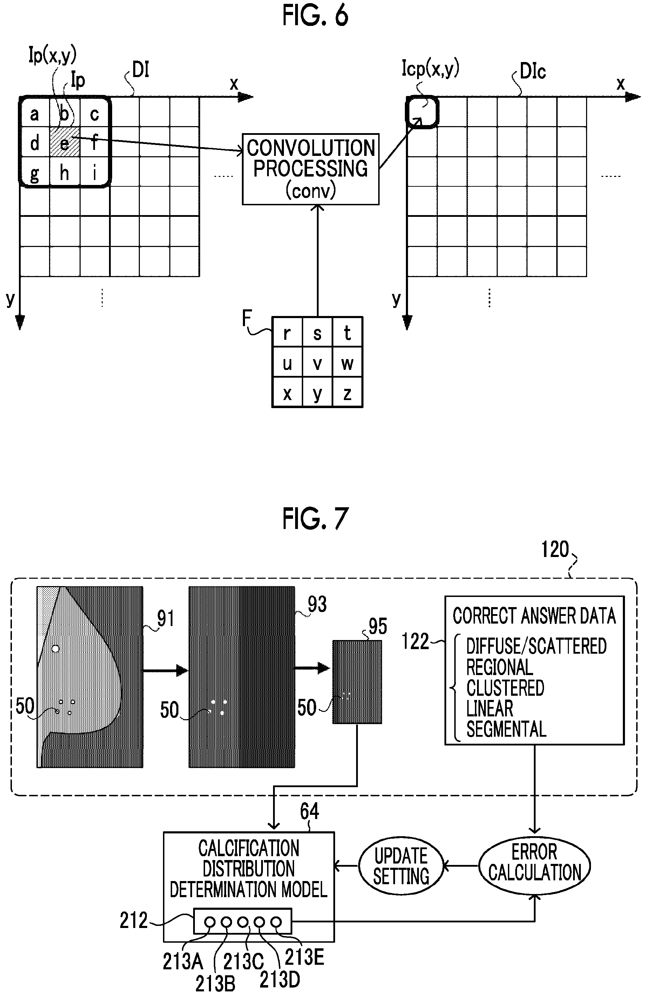

Convolution processing executed in the convolutional layer 202 and the convolutional layer 206 will be described with reference to Fig. 6. As illustrated in Fig. 6, in the convolution processing, in a case in which a pixel value Ip(x,y) of a pixel of interest Ip of input data DI to "e", pixel values of surrounding adjacent pixels to "a" to "d" and "f" to "i", and coefficients of a 3 × 3 filter F to "r" to "z", a pixel value Icp(x,y) of a pixel Icp of output data DIc, which is a result of the convolution operation related to the pixel of interest Ip, is obtained, for example, according to the following expression (1). It should be noted that the coefficients of the filter F corresponds to a weight indicating the strength of the connection between nodes of the front and rear layers.

$$Icp(x,y) = a \times z + b \times y + c \times x + d \times w + e \times v + f \times u + g \times t + h \times s + i \times r \dots (1)$$

In the convolution processing, the convolution operation described above is performed on each pixel to output the pixel value Icp(x,y) corresponding to each pixel of interest Ip. In this way, the output data DIc having the pixel values Icp(x,y) arranged in a two-dimensional manner is output. One output data DIc is output to one filter F. In a case in which a plurality of filters F having different types are used, the output data DIc is output for each filter F. The filter F means a neuron (node) of the convolutional layer, and the number of features that can be extracted from one input data DI in the convolutional layer is the number of filters F because the extractable feature is determined for each filter F.

In the pooling layer 204 and the pooling layer 208, the pooling processing of reducing the original image while leaving the features is performed. In other words, in the pooling layer 204 and the pooling layer 208, the pooling processing of reducing the image size by reducing the resolution of the input image by selecting a local representative value is performed. For example, in a case in which the pooling processing of selecting the representative value from the blocks of pixels of 2 × 2 is performed by shifting the stride by "1", that is, one pixel, a reduction image reduced to half the size of the input image is output.

In the present embodiment, as illustrated in Fig. 5, the convolutional layer 202 and the convolutional layer 206, and the pooling layer 204 and the pooling layer 208 are arranged in the order of the convolutional layer 202, the pooling layer 204, the convolutional layer 206, and the pooling layer 208 from the side closer to the input layer 200.

As illustrated in Fig. 5, the convolutional layer 202 applies a 3 × 3 filter F1 to the input (propagated) image and performs the above-described convolution operation to output an image feature map cmp1 in which the feature of the input image is extracted and the pixel values are arranged in a two-dimensional manner. In this way, the number of the image feature maps cmp1 is the number corresponding to the type of the filter F1.

The pooling layer 204 performs the pooling processing of selecting the representative value from the block of pixels of 2 × 2 on the image feature map cmp1, to output a plurality of image feature maps cmp2 in which the size of the image feature map cmp1 is reduced to 1/4 (the size in the vertical and horizontal directions is 1/2).

As in the convolutional layer 202, the convolutional layer 206 applies a 3 × 3 filter F2 and performs the above-described convolution operation to output a plurality of image feature map cmp3 in which the feature of the input image feature map cmp2 is extracted and the pixel values are arranged in a two-dimensional manner.

As in the pooling layer 204, the pooling layer 208 performs the pooling processing of selecting the representative value from the block of pixels of 2 × 2 on the image feature map cmp3, to output a plurality of image feature maps cmp4 in which the size of the image feature map cmp3 is reduced to 1/4 (the size in the vertical and horizontal directions is 1/2).

The flat layer 210 behind the intermediate layer 201 is rearranged in a state in which the numerical value of the data itself is left as the image feature map cmp4. As illustrated in Fig. 5, the three-dimensional data represented by a plurality of image feature maps cmp4 is rearranged as one-dimensional data. As illustrated in Fig. 5, the value of each node 211 included in the flat layer 210 corresponds to the pixel value of each pixel of the plurality of image feature maps cmp4.

The output layer 212 is a fully connected layer to which all the nodes 211 are connected, and includes a node 213A corresponding to the determination that the type of the distribution state of the calcification is "diffuse or scattered", a node 213B corresponding to the determination as being "regional", a node 213C corresponding to the determination as being "clustered", a node 213D corresponding to the determination as being "linear", and a node 213E corresponding to the determination as being "segmental". The output layer 212 uses a softmax function which is an example of an activation function, to output a probability corresponding to the determination that the type of the distribution state of the calcification corresponding to the node 213A is diffuse or scattered, a probability corresponding to the determination that the type of the distribution state of the calcification corresponding to the node 213B is regional, a probability corresponding to the determination that the type of the distribution state of the calcification corresponding to the node 213C is clustered, a probability corresponding to the determination that the type of the distribution state of the calcification corresponding to the node 213D is linear, and a probability corresponding to the determination that the type of the distribution state of the calcification corresponding to the node 213E is segmental. It should be noted that the calcification distribution determination model 64 may output information representing the probability of each of the nodes 213A to 213E instead of outputting a label such as "regional" or "clustered" as the determination result.

As illustrated in Fig. 7, the calcification distribution determination model 64 is generated by training a machine learning model through machine learning using training data 120. The training data 120 is composed of a set of a low resolution image 95 and correct answer data 122. The low resolution image 95 is an image obtained by reducing the resolution of the calcification distribution image 93 representing the detection result obtained by detecting the calcification 50, from the radiation image 91 captured by the mammography apparatus 10. Fig. 7 schematically illustrates that the low resolution image 95 is illustrated with the image itself of the image diagram being made smaller than the calcification distribution image 93.

The correct answer data 122 is information representing which of "diffuse or scattered", "regional", "clustered", "linear", or "segmental" is the type of the distribution state of the calcification 50 included in the radiation image 91. In the present embodiment, a backpropagation method is used to perform the machine learning of the calcification distribution determination model 64.

In the training of the calcification distribution determination model 64, the low resolution image 95 in the training data 120 is input to the calcification distribution determination model 64. It should be noted that, in the present embodiment, the training is performed by inputting the entire low resolution image 95 is input to the calcification distribution determination model 64.

The calcification distribution determination model 64 outputs the determination result of the type of the distribution state of the calcification 50 included in the low resolution image 95, specifically, the values of the nodes 213A to 213E included in the output layer 212 of the calcification distribution determination model 64.

In a case in which the correct answer data 122 for the low resolution image 95 input to the calcification distribution determination model 64 is "diffuse or scattered", the value of the node 213A should be "1", and the values of the nodes 213B to 213E should be "0". In a case in which the correct answer data 122 for the low resolution image 95 input to the calcification distribution determination model 64 is "regional", the value of the node 213B should be "1", and the values of the nodes 213A and 213C to 213E should be "0". In a case in which the correct answer data 122 for the low resolution image 95 input to the calcification distribution determination model 64 is "clustered", the value of the node 213C should be "1", and the values of the nodes 213A, 213B, 213D, and 213E should be "0". In a case in which the correct answer data 122 for the low resolution image 95 input to the calcification distribution determination model 64 is "linear", the value of the node 213D should be "1", and the values of the nodes 213A to 213C and 213E should be "0". In a case in which the correct answer data 122 for the low resolution image 95 input to the calcification distribution determination model 64 is "segmental", the value of the node 213E should be "1", and the values of the nodes 213A to 213D should be "0".

Therefore, a difference (error) between the values of the nodes 213A to 213E output from the calcification distribution determination model 64 and the values that should be taken by the nodes 213A to 213E corresponding to the correct answer data 122 is calculated. Then, the weight of each neuron is updated and set to reduce the error from the output layer 212 to the input layer 200 by using an error propagation method in accordance with the error, and the calcification distribution determination model 64 is updated in accordance with the update setting.

In the training of the calcification distribution determination model 64, the series of processing of inputting the low resolution image 95 in the training data 120 to the calcification distribution determination model 64, outputting the respective values of the nodes 213A to 213E included in the output layer 212 from the calcification distribution determination model 64, error calculation based on the respective values of the nodes 213A to 213E and the correct answer data 122, update setting of the weight, and updating the calcification distribution determination model 64 is repeatedly performed.

It should be noted that the training of the calcification distribution determination model 64 may be performed by the image processing apparatus 16, or may be performed by an external training apparatus, and the image processing apparatus 16 may acquire the trained calcification distribution determination model 64 and store the acquired calcification distribution determination model 64 in the storage unit 62.

Meanwhile, the display unit 70 of the image processing apparatus 16 displays the radiation image or various types of information. The display unit 70 is not particularly limited, and various displays and the like may be used. In addition, the operation unit 72 is used by the doctor to input instructions for the diagnosis of a lesion of the breast using the radiation image, the user to input various types of information, or the like. The operation unit 72 is not particularly limited, and examples of the operation unit 72 include various switches, a touch panel, a touch pen, and a mouse. It should be noted that the display unit 70 and the operation unit 72 may be integrated into a touch panel display.

The communication I/F unit 74 transmits and receives various types of information to and from the console 12 via the network 17 using wireless communication or wired communication.

Further, a function of outputting the determination result obtained by determining the type of the distribution state of the calcification of the breast in the radiation image captured by the mammography apparatus 10 via the image processing apparatus 16 will be described. It should be noted that, in the present embodiment, as an example, a case will be described in which the radiation image as the determination target of the type of the distribution state of the calcification is the normal two-dimensional image.

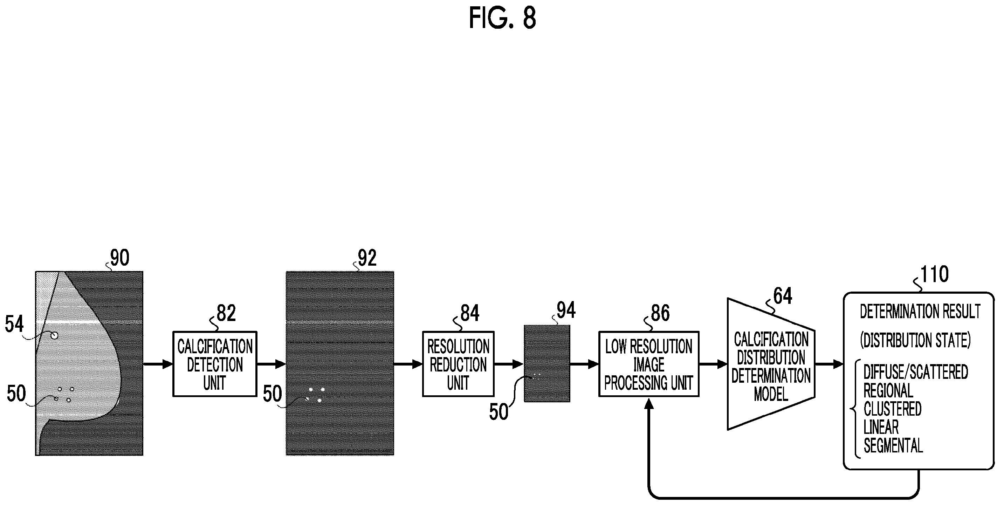

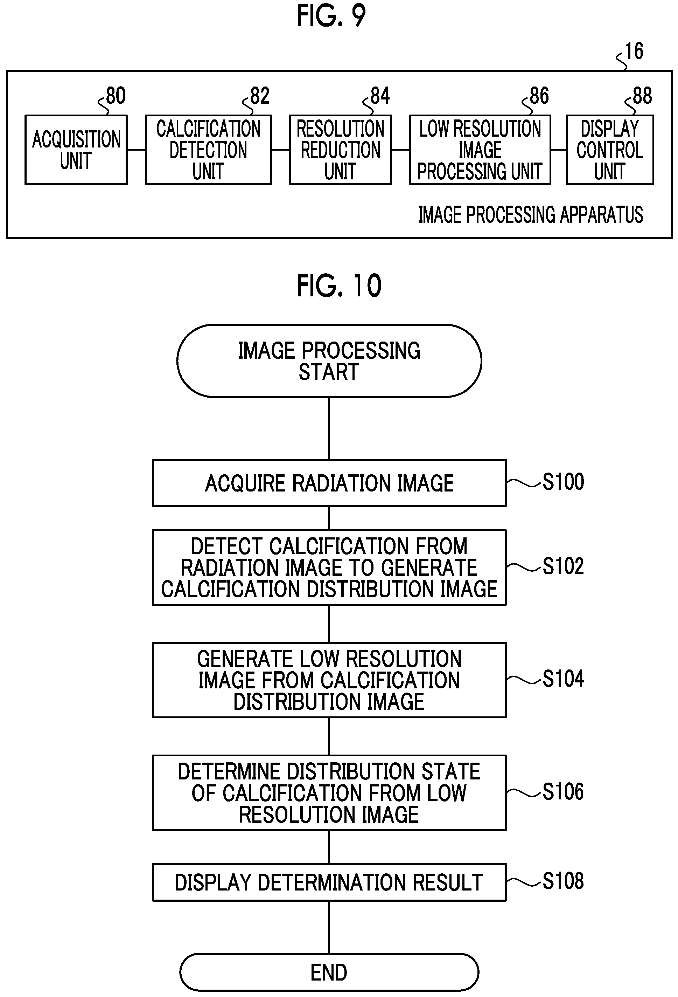

Fig. 8 is a schematic diagram illustrating an outline of a flow of determining the type of the distribution state of the calcification in the image processing apparatus 16 according to the present embodiment. Fig. 9 is a functional block diagram illustrating an example of the configuration of the image processing apparatus 16 according to the present embodiment. As illustrated in Fig. 9, the image processing apparatus 16 comprises an acquisition unit 80, a calcification detection unit 82, a resolution reduction unit 84, a low resolution image processing unit 86, and a display control unit 88. As an example, in the image processing apparatus 16 according to the present embodiment, the CPU 60A of the control unit 60 executes the image processing program 63 stored in the storage unit 62, and the CPU 60A functions as the acquisition unit 80, the calcification detection unit 82, the resolution reduction unit 84, the low resolution image processing unit 86, and the display control unit 88.

The acquisition unit 80 has a function of acquiring a radiation image 90 of the breast captured by the mammography apparatus 10. Specifically, the acquisition unit 80 acquires image data representing the radiation image 90 captured by the radiation detector 20 of the mammography apparatus 10 via the communication I/F unit 46 and the communication I/F unit 74. The acquisition unit 80 outputs the acquired radiation image 90 to the calcification detection unit 82.

The calcification detection unit 82 has a function of detecting the calcification 50 from the radiation image 90 to generate a calcification distribution image 92 representing the type of the distribution state of the calcification 50. As an example, as the calcification distribution image 92 according to the present embodiment, the calcification 50 is detected from the radiation image 90 by using a rule-based calcification detection model. Specifically, the calcification detection unit 82 sets a region of interest (ROI) for each pixel of the radiation image 90, and performs the detection based on a dispersion value $\sigma_{calc}^2$ derived for the ROI by the following expression (2).

$$\text{Threshold} < \sigma_{calc}^2 \ ... \ (2)$$

It should be noted that, in the present embodiment, Threshold in the above expression (2) is a threshold value for distinguishing between the noise and the calcification. As an example, in the present embodiment, Threshold is defined based on the size of the calcified structure in an American College of Radiology (ACR) phantom, and as the value of Threshold, a dispersion value at which calcification of 1.00 mm can be detected is calculated for each device and used.

It should be noted that Threshold may be adjusted in depending on the mammary gland amount. In a case in which the mammary gland amount of the breast is large, the structures of the mammary glands overlap each other and are difficult to see, and thus the value of Threshold may be increased. It should be noted that the method in which the calcification detection unit 82 derives the mammary gland amount from the radiation image 90 is not particularly limited. Specifically, the calcification detection unit 82 derives a mammary gland content representing a content of the mammary glands in a thickness direction of the breast, which is an irradiation direction of the radiation R, as the mammary gland amount for each pixel of the radiation image 90. In a case in which there are no mammary glands and the breast consists of only fat, the mammary gland content is "0", and the mammary gland content is larger as a mammary gland density value is higher. For example, the calcification detection unit 82 can derive the mammary gland content based on the pixel values of a region that does not include the breast in the radiation image 90, that is, a so-called blank region, the pixel values of pixels corresponding to fat, the pixel values of the pixels for which the mammary gland content is derived, and an average attenuation coefficient ratio between the mammary gland and fat (an average attenuation coefficient of the mammary gland/an average attenuation coefficient of fat).

As illustrated in Fig. 8, the calcification detection unit 82 generates the calcification distribution image 92 including the information on the type of the distribution state of the calcification detected from the radiation image 90 in this way. The breast of the radiation image 90 illustrated in Fig. 8 includes the calcification 50 and a tumor 54, which is another lesion. The calcification detection unit 82 detects the calcification 50 to generate the calcification distribution image 92. The calcification distribution image 92 is a black-and-white binary image in which the calcification is represented by "1" and other regions are represented by "0". As illustrated in Fig. 8, only the calcification 50 appears as a white image in the calcification distribution image 92. It should be noted that the calcification distribution image 92 is not limited to the binary image and may be, for example, a grayscale image in which the calcification is illustrated stepwise from white to black. In addition, for example, a mask image in which a region other than the calcification 50 is masked may be used. The calcification detection unit 82 outputs the generated calcification distribution image 92 to the resolution reduction unit 84.

The resolution reduction unit 84 has a function of generating a low resolution image 94 obtained by reducing the resolution to be lower than the resolution of the radiation image 90 from the calcification distribution image 92. It should be noted that the method in which the resolution reduction unit 84 generates the low resolution image 94 from the calcification distribution image 92 is not particularly limited. For example, the low resolution image 94 may be generated by setting a predetermined number of adjacent pixels in the calcification distribution image 92 to one pixel. In addition, in this case, any of an average value, a maximum value, or a minimum value of the pixel values of the predetermined number of pixels may be used as the pixel value of one pixel in the low resolution image 94. In addition, the resolution reduction degree of the calcification distribution image 92 is not particularly limited. For example, the size of the low resolution image 94 may be determined in accordance with the size of the calcification 50 in the low resolution image 94, the size of the image to be input to the calcification distribution determination model 64, and the like, and the resolution of the calcification distribution image 92 may be reduced in accordance with the size of the low resolution image 94. The resolution reduction unit 84 outputs the generated low resolution image 94 to the low resolution image processing unit 86.

The low resolution image processing unit 86 has a function of determining the type of the distribution state of the calcification from the low resolution image 94. Specifically, the low resolution image processing unit 86 inputs the low resolution image 94 to the calcification distribution determination model 64, and acquires an output determination result 110. The low resolution image processing unit 86 outputs the determination result 110 to the display control unit 88.

The display control unit 88 has a function of performing control of displaying, on the display unit 70, the information representing the determination result 110 obtained by the low resolution image processing unit 86.

Hereinafter, the actions of the image processing apparatus 16 according to the present embodiment will be described with reference to Fig. 10. The CPU 60A executes the image processing program 63 stored in the storage unit 62, to execute the image processing illustrated in Fig. 10.

In step S100 of Fig. 10, as described above, the acquisition unit 80 acquires the radiation image 90 from the console 12.

In next step S102, the calcification detection unit 82 detects the calcification from the radiation image 90 acquired in step S100, to generate the calcification distribution image 92. As described above, the calcification detection unit 82 sets the ROI for each pixel of the radiation image 90, and detects the calcification based on the dispersion value. In addition, as described above, the calcification detection unit 82 generates the binary image representing the calcification and the other regions, as the calcification distribution image 92.

In next step S104, as described above, the resolution reduction unit 84 generates the low resolution image 94 from the calcification distribution image 92 generated in step S102.

In next step S106, as described above, the low resolution image processing unit 86 determines the type of the distribution state of the calcification from the low resolution image 94 generated in step S104. As described above, the low resolution image processing unit 86 inputs the low resolution image 94 to the calcification distribution determination model 64, and acquires the output determination result 110.

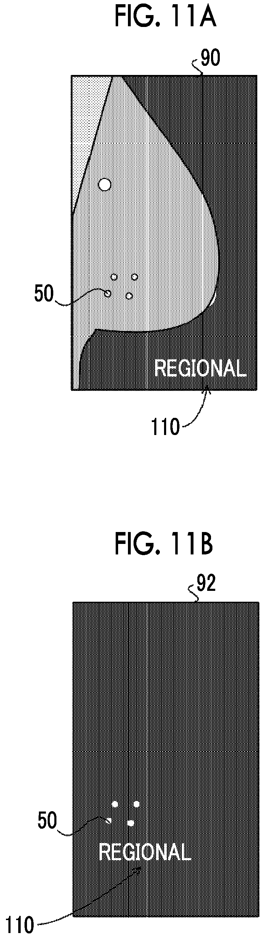

In next step S108, the display control unit 88 performs control of displaying, on the display unit 70, the determination result of the type of the distribution state of the calcification in step S106. It should be noted that the display form in which the display control unit 88 displays, on the display unit 70, the type of the distribution state of the calcification is not particularly limited. For example, a character representing any of "diffuse or scattered", "regional", "clustered", "linear", or "segmental" may be displayed on the display unit 70 as the determination result 110. In addition, for example, as illustrated in Fig. 11A, the determination result 110 may be displayed along with the radiation image 90 acquired in step S100. In addition, for example, as illustrated in Fig. 11B, the determination result 110 may be displayed along with the calcification distribution image 92 generated in step S102. It should be noted that, in the examples illustrated in Figs. 11A and 11B, the form has been illustrated in which the determination result 110 is superimposed on each of the radiation image 90 and the calcification distribution image 92, but the present disclosure is not limited to the present form, and an embodiment may be adopted in which the determination result 110 is displayed outside the radiation image 90 and the calcification distribution image 92. In addition, the calcification 50 in each of the radiation image 90 and the calcification distribution image 92 may be emphasized and displayed by changing a color or the like.

In a case in which the processing of step S108 ends, the image processing illustrated in Fig. 10 ends.

It should be noted that the above-described embodiment is merely an example, and various modification examples can be made. For example, the following modification examples may be adopted.

(Modification example 1)

In the present modification example, a modification example of the method of detecting the calcification via the calcification detection unit 82 will be described. The calcification detection unit 82 according to the above-described embodiment detects the calcification from the radiation image 90 by using a rule-based calcification detection model. On the other hand, the calcification detection unit 82 according to the present modification example detects the calcification from the radiation image 90 by using a learning-based calcification detection model.

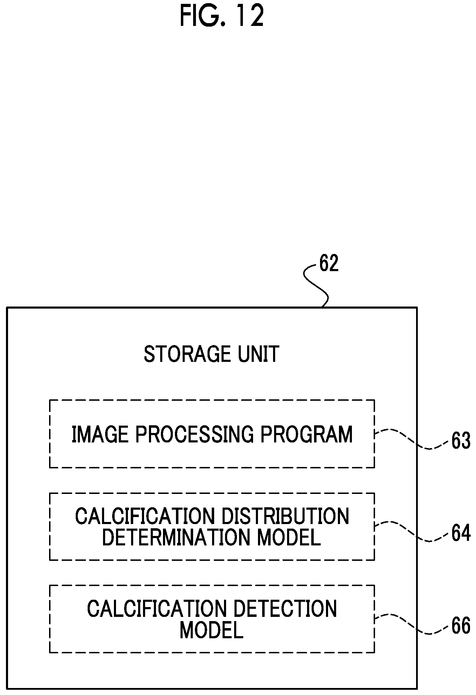

In the present embodiment, a trained model that has been trained through machine learning is used as the calcification detection model to detect the calcification from the radiation image 90 and output the calcification distribution image 92 as the detection result. Therefore, as illustrated in Fig. 12, the storage unit 62 of the image processing apparatus 16 according to the present modification example further stores a calcification detection model 66.

As the calcification detection model 66, a model obtained by subjecting the calcification to annotation and training, through machine learning, a convolutional neural network (CNN)-based segmentation model using a U-shaped neural network (U-Net) or the like, a sliding window method model to which a classifier of the CNN is sequentially applied, a machine learning model, or a multilayer perceptron (MLP) model can be used.

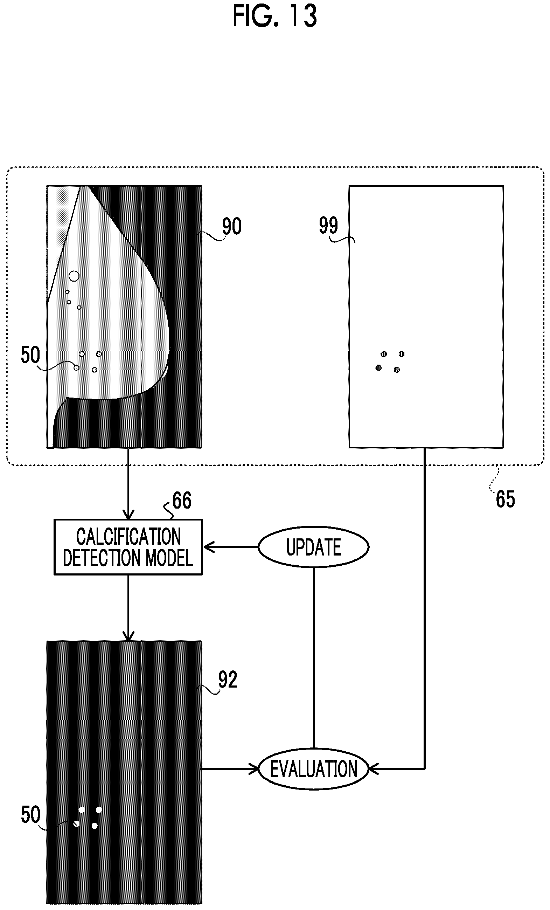

As illustrated in Fig. 13, the calcification detection model 66 is trained through machine learning using training data 65. A set of the radiation image 90 and an annotation image 99 is used as the training data 65. It should be noted that the radiation image 90 used as the training data 65 includes the radiation image 90 that does not include the calcification 50. The annotation image 99 is an image in which the calcification 50 is subjected to the annotation in advance by, for example, a person. The annotation image 99 is an image for checking, so to speak, an answer with the calcification distribution image 92 output from the calcification detection model 66 in accordance with the radiation image 90, and is compared with the calcification distribution image 92. As the detection accuracy for the calcification 50 in the calcification detection model 66 is higher, the probability that the annotation image 99 and the calcification distribution image 92 match is higher.

In a training phase of the calcification detection model 66, the radiation image 90 is input to the calcification detection model 66. As a result, the calcification distribution image 92 is output from the calcification detection model 66. The calcification distribution image 92 output from the calcification detection model 66 and the annotation image 99 are compared with each other, and the detection accuracy for the calcification 50 in the calcification detection model 66 is evaluated. The calcification detection model 66 is updated based on the evaluation result.

In the training phase, the input of the radiation image 90 to the calcification detection model 66, the output of the calcification detection model 66 to the calcification distribution image 92, the evaluation of the detection accuracy of the calcification detection model 66 by comparing the calcification distribution image 92 with the annotation image 99, and the update of the calcification detection model 66 are performed while changing the set of the radiation image 90 and the annotation image 99, and are repeated until the detection accuracy of the calcification detection model 66 reaches a desired level.

It should be noted that the training of the calcification detection model 66 may be, for example, performed by the image processing apparatus 16, or an external training apparatus may perform the training and the image processing apparatus 16 may acquire the trained calcification detection model 66 from the external training apparatus.

It should be noted that, in the image processing according to the present modification example, in step S102 of the image processing of the above-described embodiment (see Fig. 10), the calcification detection unit 82 inputs the radiation image 90 to the calcification detection model 66 stored in the storage unit 62 to cause the calcification detection model 66 to detect the calcification, and acquires the calcification distribution image 92 output from the calcification detection model 66 to generate the calcification distribution image 92.

As described above, according to the present modification example, since the model that detects only faint and indistinct calcification may be constructed by using the trained model that has been trained through machine learning, the detection accuracy can be improved by using an appropriate model.

(Modification example 2)

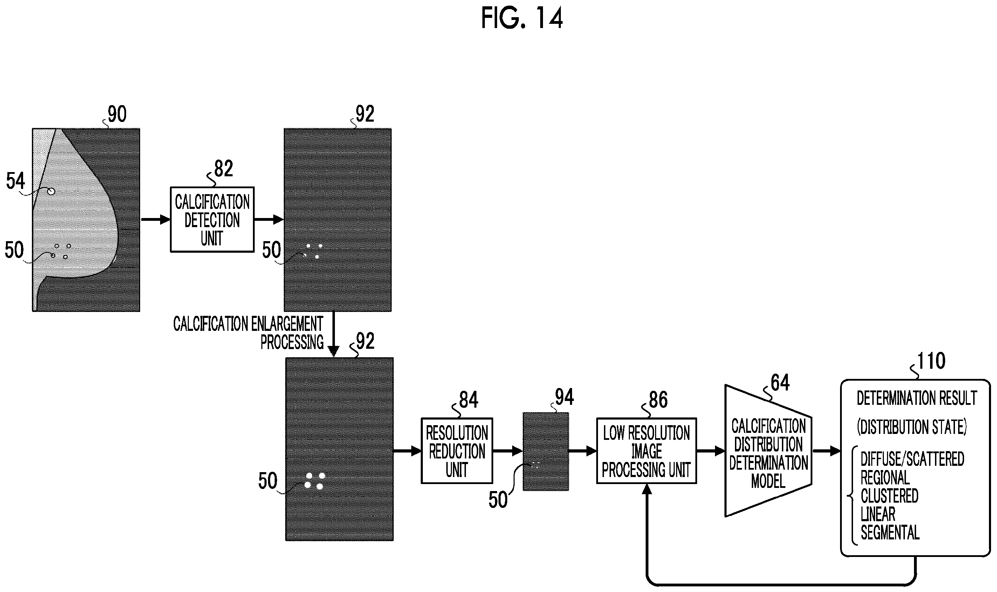

Fig. 14 illustrates a schematic diagram illustrating an outline of a flow of determining the type of the distribution state of the calcification in the image processing apparatus 16 according to the present modification example. The calcification detection unit 82 according to the present modification example enlarges the image of the calcification 50 included in the calcification distribution image 92. In addition, the resolution reduction unit 84 generates the low resolution image 94 from the calcification distribution image 92 in which the image of the calcification 50 is enlarged by the calcification detection unit 82.

By reducing the resolution of the calcification distribution image 92, the image of the calcification 50 included in the low resolution image 94 is made small as it is. Therefore, in the present modification example, the calcification detection unit 82 performs the calcification enlargement processing to enlarge the image of the calcification 50 included in the calcification distribution image 92, so that the information on the calcification 50 required for the distribution determination is not lost even in a case in which the resolution is reduced by the low resolution processing. It should be noted that the degree to which the calcification distribution image 92 enlarges the size of the calcification 50 in the image is not particularly limited. For example, the calcification detection unit 82 enlarges the image of the calcification 50 included in the calcification distribution image 92 such that the size of the image of the calcification 50 included in the radiation image 90 and the size of the image of the calcification 50 included in the low resolution image 94 are the same.

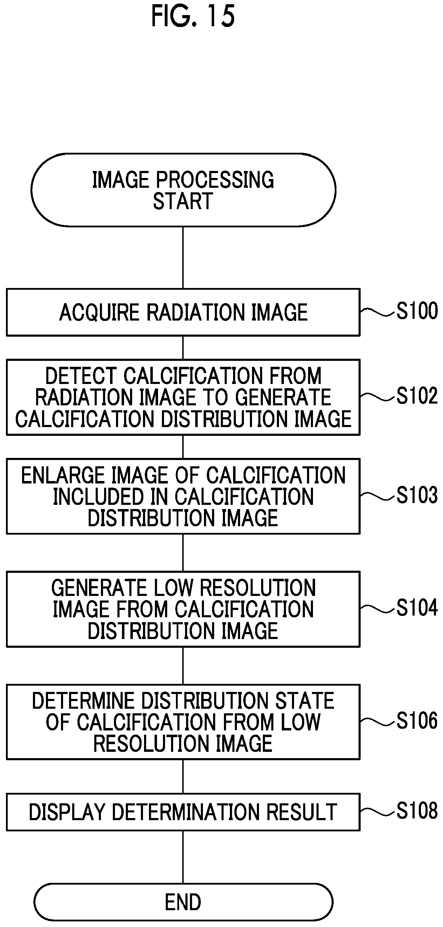

Fig. 15 illustrates a flowchart illustrating an example of a flow of image processing via the image processing apparatus 16 according to the present modification example. The image processing illustrated in Fig. 15 is different from the image processing of the above-described embodiment (see Fig. 10) in that the processing of step S103 is provided between steps S102 and S104.

In step S103 of Fig. 15, the calcification detection unit 82 performs the calcification enlargement processing to enlarge the image of the calcification 50 included in the calcification distribution image 92.

As described above, according to the present modification example, it is possible to suppress the image of the calcification 50 in the low resolution image 94 from being smaller than the image of the calcification 50 in the radiation image 90.

(Modification example 3)

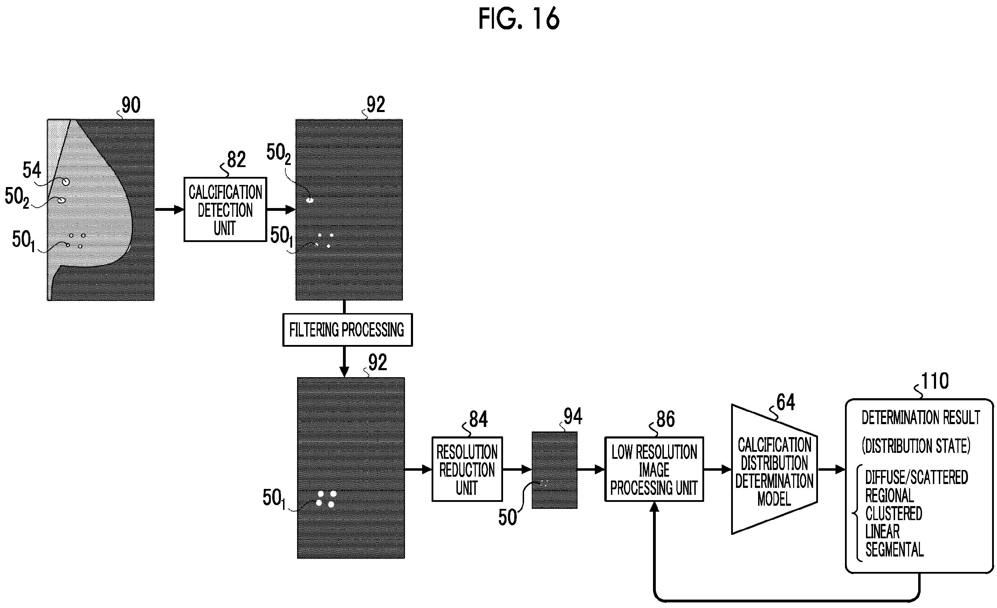

Fig. 16 illustrates a schematic diagram illustrating an outline of a flow of determining the type of the distribution state of the calcification in the image processing apparatus 16 according to the present modification example. The calcification detection unit 82 according to the present modification example performs filtering processing based on a signal value of the calcification 50 on the calcification distribution image 92. In addition, the resolution reduction unit 84 generates the low resolution image 94 from the calcification distribution image 92 subjected to the filtering processing of the calcification 50 by the calcification detection unit 82.

As described above, the calcification has various morphologies (see Fig. 4). For example, the small round calcification has a signal value larger than the signal value of the faint and indistinct calcification. Therefore, by performing the filtering processing using a threshold value corresponding to the calcification to be detected, only a desired calcification 50 can be extracted from the calcification distribution image 92. For example, in the example illustrated in Fig. 16, the radiation image 90 includes faint and indistinct calcification $50_1$ and small round calcification $50_2$. In this case, the filter for detecting the faint and indistinct calcification $50_1$ using an intermediate value of the signal value of the faint and indistinct calcification $50_1$ and the signal value of the small round calcification $50_2$ as the threshold value is applied to the calcification distribution image 92, the faint and indistinct calcification $50_1$ is extracted from the calcification distribution image 92. Then, the resolution reduction unit 84 generates the low resolution image 94 from the calcification distribution image 92 representing only the faint and indistinct calcification $50_1$.

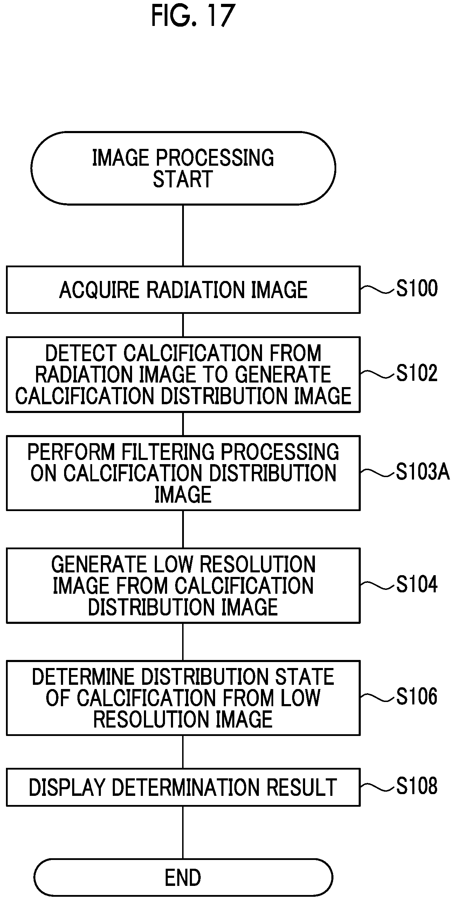

Fig. 17 illustrates a flowchart illustrating an example of a flow of image processing via the image processing apparatus 16 according to the present modification example. The image processing illustrated in Fig. 17 is different from the image processing of the above-described embodiment (see Fig. 10) in that the processing of step S103A is provided between steps S102 and S104.

In step S103A of Fig. 15, as described above, the calcification detection unit 82 performs the filtering processing on the calcification distribution image 92 and generates the calcification distribution image 92 from which only the desired calcification 50 (in Fig. 16, the faint and indistinct calcification $50_1$) is extracted.

In this way, according to the present modification example, the calcification distribution image 92 including only the calcification 50 in a desired morphology is obtained. Therefore, it is possible to determine the type of the distribution state in accordance with the morphology of the calcification 50.

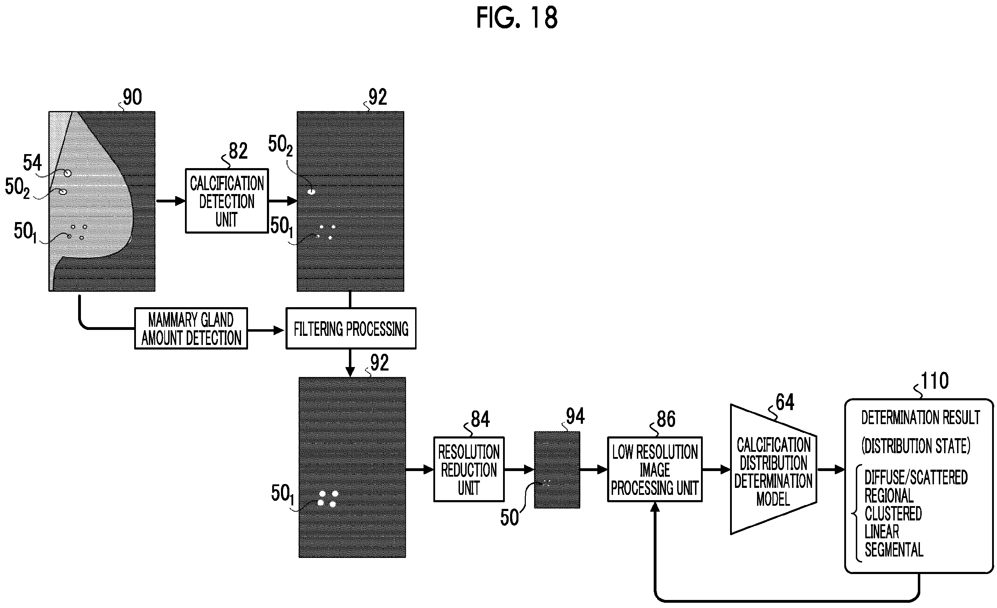

It should be noted that the threshold value in the filtering may be set based on the mammary gland amount of the breast. Fig. 18 illustrates a schematic diagram illustrating an outline of a flow of determining the type of the distribution state of the calcification in the image processing apparatus 16 in this case. For example, the threshold value in the filtering may be smaller as the mammary gland amount is larger. In this case, the calcification detection unit 82 derives the mammary gland amount of the breast from the radiation image 90. It should be noted that the method of deriving the mammary gland amount is not particularly limited, and for example, as illustrated in the above-described embodiment, the mammary gland content may be derived based on the pixel values of a region that does not include the breast in the radiation image 90, the pixel values of pixels corresponding to fat, the pixel values of the pixels for which the mammary gland content is derived, and the average attenuation coefficient ratio between the mammary gland and fat (the average attenuation coefficient of the mammary gland/the average attenuation coefficient of fat).

The calcification detection unit 82 derives the mammary gland amount from the radiation image 90 before performing the filtering processing. For example, in a case in which the derived mammary gland amount exceeds a predetermined mammary gland threshold value, the calcification detection unit 82 changes the threshold value in the filtering to a threshold value that is determined to be used in a case in which the mammary gland amount is large, and then performs the filtering processing on the calcification distribution image 92 using the changed threshold value. Further, for example, a correspondence relationship between the mammary gland amount and the threshold value used for the filtering is determined in advance, the calcification detection unit 82 specifies the threshold value corresponding to the derived mammary gland amount based on the correspondence relationship, and performs the filtering processing on the calcification distribution image 92 using the specified threshold value.

In this way, by performing the filtering processing using the threshold value corresponding to the mammary gland amount, the calcification distribution image 92 including only the calcification 50 in the desired morphology is obtained with high accuracy.

(Modification example 4)

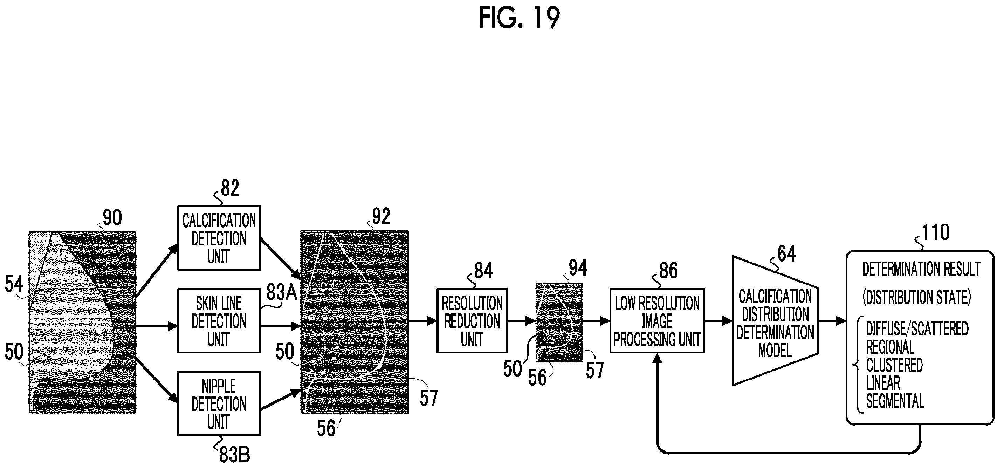

Fig. 19 illustrates a schematic diagram illustrating an outline of a flow of determining the type of the distribution state of the calcification in the image processing apparatus 16 according to the present modification example. The calcification distribution image 92 according to the present modification example includes a skin line 56 and a nipple 57 of the breast in addition to the calcification 50.

As illustrated in Fig. 19, the image processing apparatus 16 according to the present modification example further comprises a skin line detection unit 83A and a nipple detection unit 83B.

The skin line detection unit 83A detects the skin line 56 of the breast from the radiation image 90. It should be noted that the method in which the skin line detection unit 83A detects the skin line 56 of the breast is not particularly limited, and a known technique can be applied. For example, JP2008-086389A describes a method of examining the density of the radiation image 90, detecting the position at which a density level difference is equal to or greater than a predetermined value, and defining a set of pixels having the density level difference equal to or greater than the predetermined value as the skin line. In addition, for example, JP2010-051456A discloses a method of dividing the radiation image obtained by imaging the breast into a breast region and a blank region based on the density of each pixel of the radiation image 90 and connecting the pixels which are the boundary points between the breast region and the blank region to perform the generation.

The skin line detection unit 83A superimposes the detected skin line 56, strictly speaking, an image of the skin line 56 on the calcification distribution image 92.

The nipple detection unit 83B detects the nipple 57 of the breast from the radiation image 90. It should be noted that the method in which the nipple detection unit 83B detects the nipple 57 is not particularly limited, and a known technique can be applied. For example, a portion of the skin line of the breast that protrudes into the blank region may be detected as the nipple.

The nipple detection unit 83B superimposes the detected nipple 57, strictly speaking, an image representing the nipple 57 on the calcification distribution image 92.

The resolution reduction unit 84 generates the low resolution image 94 from the calcification distribution image 92 including the calcification 50, the skin line 56, and the nipple 57.

In this way, according to the present modification example, since the skin line 56 and the nipple 57 are included in the calcification distribution image 92, the type of the distribution state of the calcification 50 can be determined with reference to the skin line 56 and the nipple 57. Therefore, it is possible to easily determine the type of the distribution state of the calcification 50, and it is possible to improve the determination accuracy.

(Modification example 5)

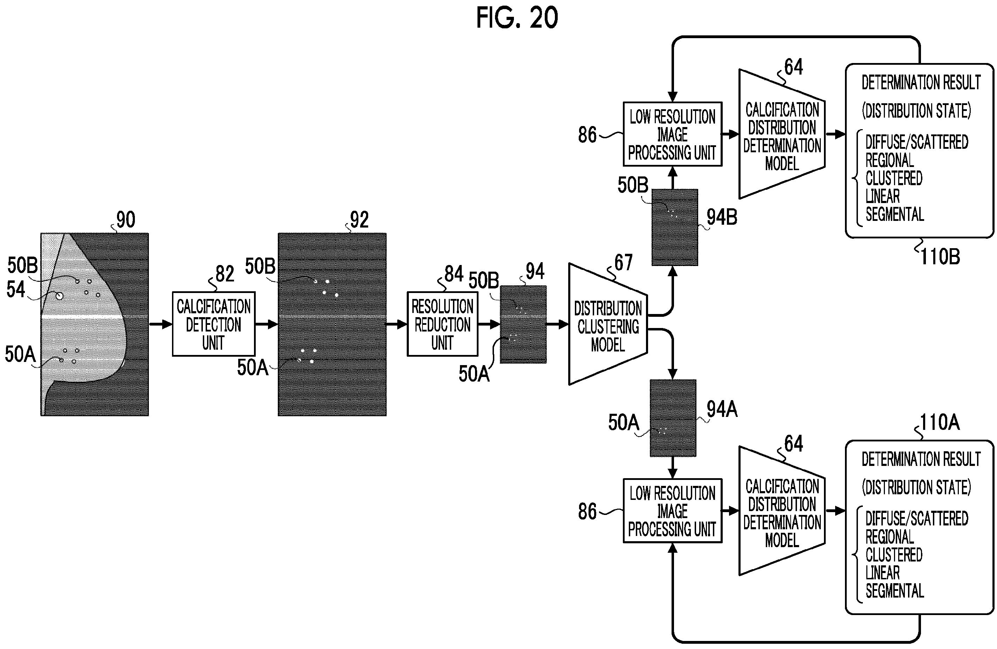

Fig. 20 illustrates a schematic diagram illustrating an outline of a flow of determining the type of the distribution state of the calcification in the image processing apparatus 16 according to the present modification example.

The radiation image 90 illustrated in Fig. 20 includes calcification 50A and calcification 50B included in the different distributions, respectively, and the calcification distribution image 92 includes the calcification 50A and the calcification 50B. In a case in which the calcification distribution image 92 includes a plurality of distributions, the low resolution image processing unit 86 according to the present modification example determines the type of the distribution state for each distribution.

Therefore, the low resolution image processing unit 86 determines whether or not the plurality of distributions are included in the calcification distribution image 92 for the calcification 50, and generates the low resolution image 94 for each distribution from the low resolution image 94 in a case in which the plurality of distributions are included. It should be noted that the method in which the low resolution image processing unit 86 determines whether or not the plurality of distributions are included in the calcification distribution image 92 is not particularly limited. In the example illustrated in Fig. 20, a distribution clustering model 67 that detects the distribution of the calcification 50 by performing clustering processing on the calcification 50 (50A and 50B) included in the low resolution image 94 may be applied to determine whether or not the plurality of distributions are included in the low resolution image 94, to determine whether or not the plurality of distributions are included in the calcification distribution image 92.

In the example illustrated in Fig. 20, the low resolution image processing unit 86 applies the distribution clustering model 67 to the low resolution image 94 to generate a low resolution image 94A including the calcification 50A and to generate a low resolution image 94B including the calcification 50B. In addition, the low resolution image processing unit 86 applies the calcification distribution determination model 64 to the low resolution image 94A to derive a determination result 110A, and applies the calcification distribution determination model 64 to the low resolution image 94B to derive a determination result 110B. It should be noted that, as the distribution clustering model 67, for example, a hierarchical clustering method, such as a centroid method, can be applied. With the present method, the processing of grouping the calcification groups having a short distance therebetween into the same cluster is recursively performed to create a dendrogram. In a case in which the distance between the clusters is equal to or greater than a threshold value, the clusters are separated as another calcification set. Alternatively, the method of determining the cluster by applying an x-means method, which is an unsupervised learning method, can also be considered.

(Modification example 6)

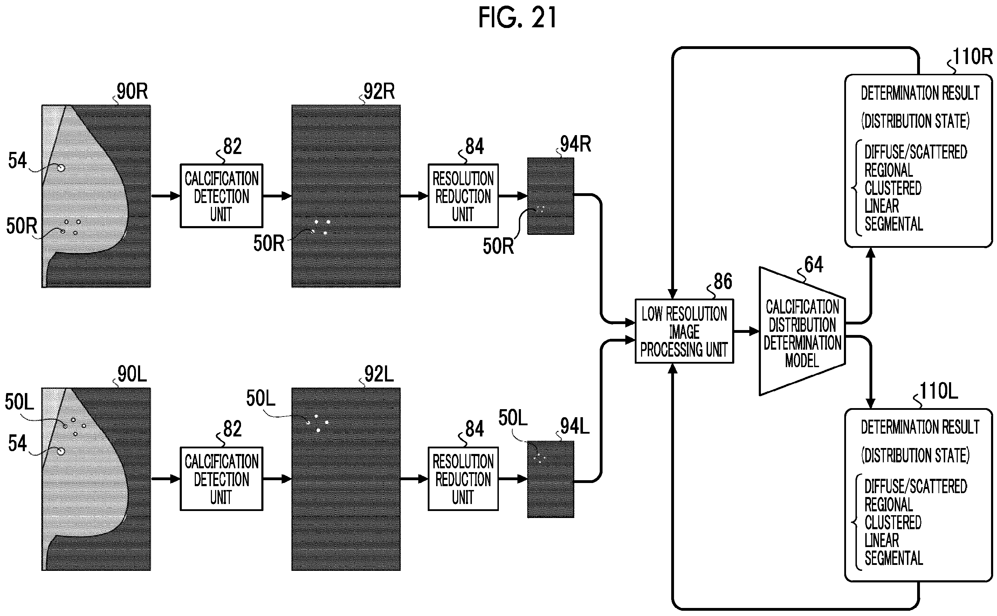

Fig. 21 illustrates a schematic diagram illustrating an outline of a flow of determining the type of the distribution state of the calcification in the image processing apparatus 16 according to the present modification example. In the present modification example, the calcification distribution determination model 64 is applied to a low resolution image 94L generated based on a radiation image 90L of a left breast of the same subject and a low resolution image 94R generated based on a radiation image 90R of a right breast, to derive a determination result 110L for the left breast and a determination result 110R for the right breast.

In the example illustrated in Fig. 21, the calcification detection unit 82 detects calcification 50L from the radiation image 90L of the left breast to generate a calcification distribution image 92L. The resolution reduction unit 84 generates the low resolution image 94L from the calcification distribution image 92L. In addition, the calcification detection unit 82 detects calcification 50R from the radiation image 90R of the right breast to generate a calcification distribution image 92R. The resolution reduction unit 84 generates the low resolution image 94R from the calcification distribution image 92R. The low resolution image processing unit 86 inputs the low resolution image 94L and the low resolution image 94R to the calcification distribution determination model 64, and acquires the output determination result 110R and determination result 110L.

In this way, according to the present modification example, the type of the distribution state in each of the left breast and the right breast can be determined by comparing the distribution of the calcification 50L in the left breast with the distribution of the calcification 50R in the right breast. Therefore, according to the present modification example, it is possible to determine the type of the distribution state of the calcification of the breast with higher accuracy.

(Modification example 7)

In the present modification example, a form will be described in which the image processing apparatus 16 estimates a category to which the calcification 50 belongs, based on the type of the distribution state of the calcification 50 and the type of the shape of the calcification 50. It should be noted that the category in the present modification example corresponds to an example of a degree of malignancy according to the present disclosure.

The low resolution image processing unit 86 according to the present modification example further has a function of determining the type of the shape of the calcification 50 from the low resolution image 94.

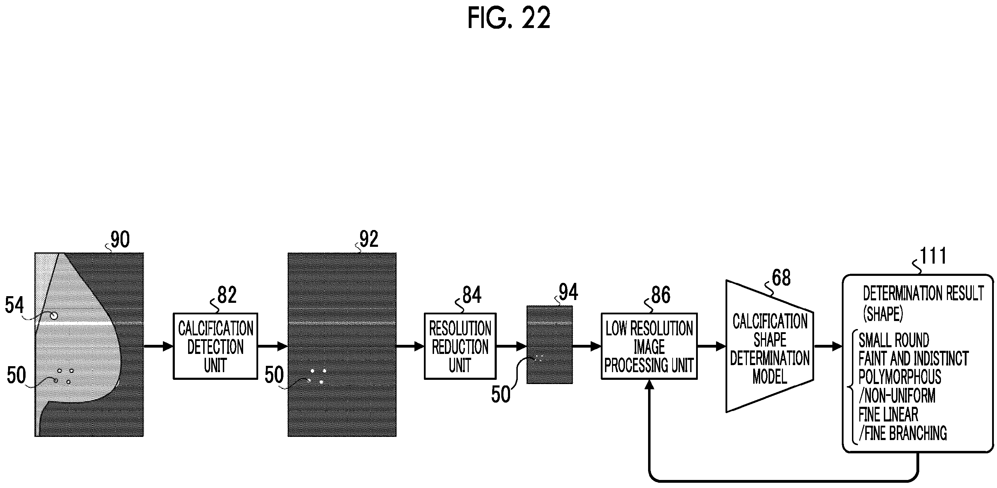

Fig. 22 illustrates a schematic diagram illustrating an outline of a flow of determining the type of the shape of the calcification in the low resolution image processing unit 86 of the image processing apparatus 16 according to the present modification example.

Since the processing until the low resolution image 94 is input to the low resolution image processing unit 86 is as described above, the description thereof will be omitted. In a case of determining the type of the shape of the calcification 50, the low resolution image processing unit 86 inputs the low resolution image 94 to a calcification shape determination model 68 to acquire an output determination result 111.

The calcification shape determination model 68 is a model that outputs the determination result 111 obtained by determining the type of the shape of the calcification 50, in a case in which the low resolution image 94 including the calcification 50 is input. As an example, the calcification shape determination model 68 according to the present modification example determines which of the above-described morphologies of the calcification, that is, "small round", "faint and indistinct", "polymorphous or non-uniform", or "fine linear or fine branching" is the type of the shape of the calcification 50.

For example, the calcification shape determination model 68 can use a convolutional neural network (CNN) (see Fig. 5) that has been subjected to machine learning through deep learning using the training data, similarly to the calcification distribution determination model 64.

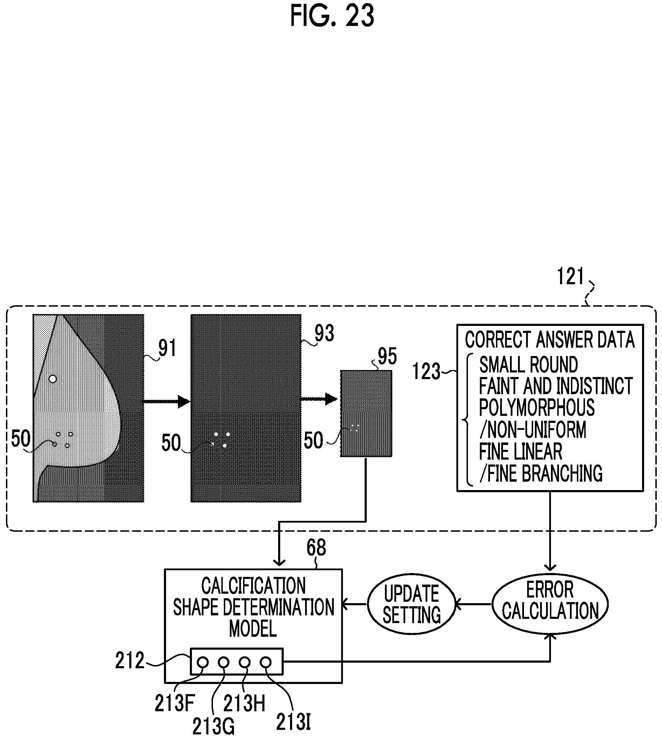

As illustrated in Fig. 23, the calcification shape determination model 68 is generated by training a machine learning model through machine learning using training data 121. The training data 121 is composed of a set of the low resolution image 95 and correct answer data 123. As described above, the low resolution image 95 is an image obtained by reducing the resolution of the calcification distribution image 93 representing the detection result obtained by detecting the calcification 50, from the radiation image 91 captured by the mammography apparatus 10.

The correct answer data 123 is information representing which of "small round", "faint and indistinct", "polymorphous or non-uniform", or "fine linear or fine branching" is the type of the shape of the calcification 50 included in the radiation image 91. It should be noted that, specifically, the correct answer data 123 is information representing the type of the shape of the calcification 50 included in the low resolution image 95 corresponding to the radiation image 91. In addition, in a case in which a plurality of pieces of calcification 50 are included in the low resolution image 95, information representing the type of the shape estimated that the degree of malignancy is highest among the types of the shapes of the plurality of pieces of calcification 50 is used as the correct answer data 123. Specifically, in a case in which the plurality of pieces of calcification 50 included in the low resolution image 95 include the calcification 50 of which the type of the shape is "fine linear or fine branching", the correct answer data 123 for the low resolution image 95 is information representing "fine linear or fine branching". In addition, in a case in which the plurality of pieces of calcification 50 included in the low resolution image 95 include the calcification 50 of which the type of the shape is "polymorphous or non-uniform" and do not include the calcification 50 of which the type of the shape is "fine linear or fine branching", the correct answer data 123 for the low resolution image 95 is information representing "polymorphous or non-uniform". In a case in which the plurality of pieces of calcification 50 included in the low resolution image 95 include the calcification 50 of which the type of the shape is "faint and indistinct" and do not include the calcification 50 of which the type of the shape is "fine linear or fine branching" and the calcification 50 of which the type of the shape is "polymorphous or non-uniform", the correct answer data 123 for the low resolution image 95 is information representing "faint and indistinct". In a case in which the plurality of pieces of calcification 50 included in the low resolution image 95 include the calcification 50 of which the shape type is "small round" and do not include the calcification 50 of which the shape type is "fine linear or fine branching", the calcification 50 of which the shape type is "polymorphous or non-uniform", and the calcification 50 of which the shape type is "faint and indistinct", the correct answer data 123 for the low resolution image 95 is information representing "small round". In the present embodiment, a backpropagation method is used to perform the machine learning of the calcification shape determination model 68.

In the training of the calcification shape determination model 68, the low resolution image 95 in the training data 121 is input to the calcification shape determination model 68. It should be noted that, in the present embodiment, the training is performed by inputting the entire low resolution image 95 to the calcification shape determination model 68.

The calcification shape determination model 68 outputs the determination result of the type of the shape of the calcification 50 included in the low resolution image 95, specifically, the values of the nodes 213F to 213I included in the output layer 212 of the calcification shape determination model 68. In a case in which the correct answer data 123 for the low resolution image 95 input to the calcification shape determination model 68 is "small round", the value of the node 213F should be "1", and the values of the nodes 213G to 213I should be "0". In a case in which the correct answer data 123 for the low resolution image 95 input to the calcification shape determination model 68 is "faint and indistinct", the value of the node 213G should be "1", and the values of the nodes 213F, 213H, and 213I should be "0". In a case in which the correct answer data 123 for the low resolution image 95 input to the calcification shape determination model 68 is "polymorphous or non-uniform", the value of the node 213H should be "1", and the values of the nodes 213F, 213G, and 213I should be "0". In a case in which the correct answer data 123 for the low resolution image 95 input to the calcification shape determination model 68 is "fine linear or fine branching", the value of the node 213I should be "1", and the values of the nodes 213F to 213H should be "0".

Therefore, a difference (error) between the values of the nodes 213F to 213I output from the calcification shape determination model 68 and the values that should be taken by the nodes 213F to 213I corresponding to the correct answer data 123 is calculated. Then, the weight of each neuron is updated and set to reduce the error from the output layer 212 to the input layer 200 by using an error propagation method in accordance with the error, and the calcification shape determination model 68 is updated in accordance with the update setting.

In the training of the calcification shape determination model 68, the series of processing of inputting the low resolution image 95 in the training data 121 to the calcification shape determination model 68, outputting the respective values of the nodes 213F to 213I included in the output layer 212 from the calcification shape determination model 68, error calculation based on the respective values of the nodes 213F to 213I and the correct answer data 123, update setting of the weight, and updating the calcification shape determination model 68 is repeatedly performed.

It should be noted that the training of the calcification shape determination model 68 may be performed by the image processing apparatus 16, or may be performed by an external training apparatus, and the image processing apparatus 16 may acquire the trained calcification shape determination model 68 and store the acquired calcification shape determination model 68 in the storage unit 62.

In this way, the low resolution image processing unit 86 estimates the category to which the calcification 50 belongs based on the determination result 111 of the type of the shape of the calcification output from the calcification shape determination model 68 and the determination result 110 of the type of the distribution state of the calcification output from the calcification distribution determination model 64. As an example, the low resolution image processing unit 86 according to the present modification example estimates the category to which the calcification 50 belongs based on the determination result 111 of the type of the shape of the calcification and the determination result 110 of the type of the distribution state of the calcification with reference to the correspondence relationship illustrated in Fig. 4 described above.

The low resolution image processing unit 86 outputs the determination results 110 and 111 and the information representing the estimated category to the display control unit 88.

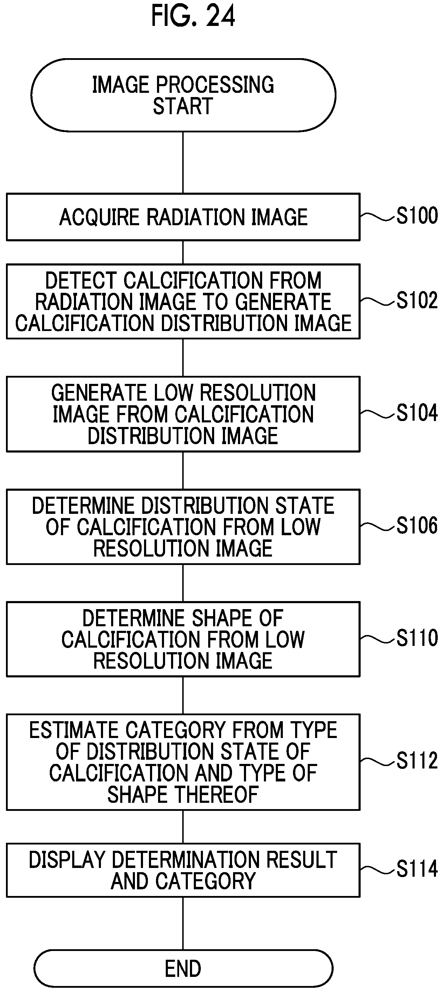

Fig. 24 illustrates a flowchart illustrating an example of a flow of image processing via the image processing apparatus 16 according to the present modification example. The image processing illustrated in Fig. 24 is different from the image processing of the above-described embodiment (see Fig. 10) in that the pieces of processing of steps S110 to S114 are provided instead of step S108.

In step S110 of Fig. 24, the low resolution image processing unit 86 determines the type of the shape of the calcification from the low resolution image 94 generated in step S104. As described above, the low resolution image processing unit 86 inputs the low resolution image 94 to the calcification shape determination model 68, and acquires the output determination result 111.

In next step S112, the low resolution image processing unit 86 estimates the category to which the calcification belongs based on the type of the distribution state of the calcification determined in step S106 and the type of the shape of the calcification determined in step S108. As described above, the low resolution image processing unit 86 estimates the category with reference to the correspondence relationship illustrated in Fig. 4.

In next step S114, the display control unit 88 performs control of displaying, on the display unit 70, the determination result 110 of the type of the distribution state of the calcification by step S106, the determination result 111 of the type of the shape of the calcification by step S110, and the estimation result of the category by step S112. It should be noted that the display form in which the display control unit 88 displays, on the display unit 70, the type of the distribution state of the calcification, the type of the shape of the calcification, and the estimated category is not particularly limited. In a case in which step S114 ends, the image processing illustrated in Fig. 24 ends.

In this way, according to the present modification example, it is possible to estimate the category of the calcification 50 based on the type of the shape of the calcification 50 and the type of the distribution state in order to determine the type of the shape of the calcification 50 from the low resolution image 94.

It should be noted that, in the image processing illustrated in Fig. 24, the form has been illustrated in which the low resolution image processing unit 86 of the image processing apparatus 16 estimates the category to which the calcification 50 belongs, but the estimation of the category of the calcification 50 based on the type of the shape of the calcification 50 and the type of the distribution state need not be performed. For example, the doctor or the like may determine the category of the calcification 50. In this case, step S112 among the series of steps of the image processing illustrated in Fig. 24 is omitted. In addition, in step S114, the display control unit 88 need only perform control of displaying, on the display unit 70, the determination result 110 of the type of the distribution state of the calcification in step S106 and the determination result 111 of the type of the shape of the calcification in step S110. In the present form, the doctor or the like can refer to the type of the distribution state of the calcification 50 displayed on the display unit 70 and the type of the shape thereof, and to determine whether the calcification 50 is malignant or benign, determine the category, and the like from the correspondence relationship illustrated in Fig. 4.

The calcification shape determination model 68 and the training method thereof are not limited to the above-described methods. For example, the calcification shape determination model 68 may be a semantic segmentation model, and the determination of the type of the shape may be performed for each of the plurality of pieces of calcification 50 included in the low resolution image 94. In this case, the training data 121 is composed of a set of the low resolution image 95 and a correct answer label image corresponding to the low resolution image 95. As the correct answer label image in this case, an image in which the shape of calcification 50 is used as an independent class and an identifier (ID) corresponding to each shape is used as the pixel value is used. For example, in a case in which "small round", "faint and indistinct", "polymorphous or non-uniform", and "fine linear or fine branching" are associated with "1", "2", "3", and "4" as the IDs, respectively, the correct answer label image is an image in which the pixel values of the plurality of pieces of calcification 50 included in the low resolution image 95 are set to the values (1 to 4) of the IDs depending on the type of the shape.

In addition, for example, for each of the plurality of pieces of calcification 50 included in the low resolution image 95, the ROI may be set, and a correct answer label of "small round", "faint and indistinct", "polymorphous or non-uniform", and "fine linear or fine branching" may be assigned to each ROI. In this case, the training data 121 is composed of a set of the low resolution image 95, an image of the set ROI, and the correct answer label assigned to the image of the ROI.

(Modification example 8)

In the present modification example, a form will be described in which the low resolution image processing unit 86 further has a function of estimating whether the calcification 50 is benign or malignant from the low resolution image 94.

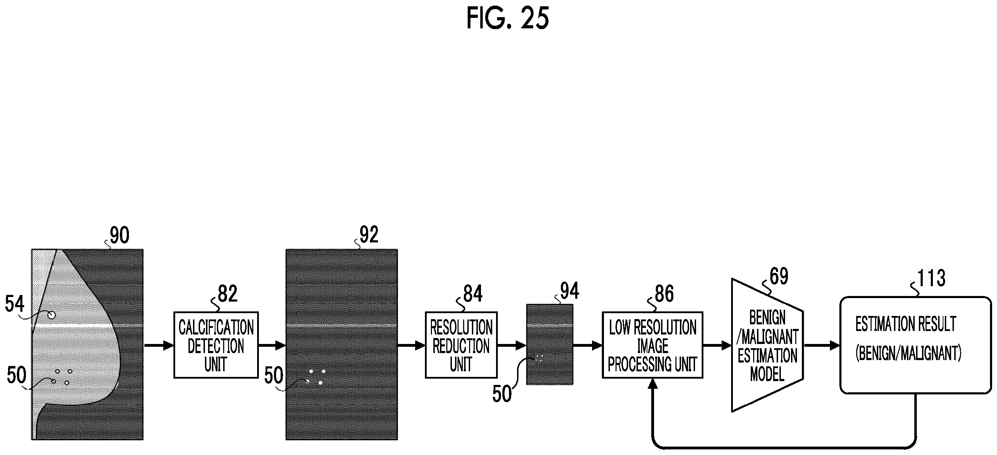

Fig. 25 is a schematic diagram illustrating an outline of a flow of estimating whether the calcification is benign or malignant in the low resolution image processing unit 86 of the image processing apparatus 16 according to the present modification example.

Since the processing until the low resolution image 94 is input to the low resolution image processing unit 86 is as described above, the description thereof will be omitted. In a case of estimating whether the calcification 50 is benign or malignant, the low resolution image processing unit 86 inputs the low resolution image 94 to the benign/malignant estimation model 69, and acquires an output estimation result 113.

The benign/malignant estimation model 69 is a model that outputs the estimation result 113 in which it is estimated whether the calcification 50 is benign or malignant, in a case in which the low resolution image 94 including the calcification 50 is input. For example, the benign/malignant estimation model 69 can use a convolutional neural network (CNN) (see Fig. 5) that has been subjected to machine learning through deep learning using the training data, similarly to the calcification distribution determination model 64.

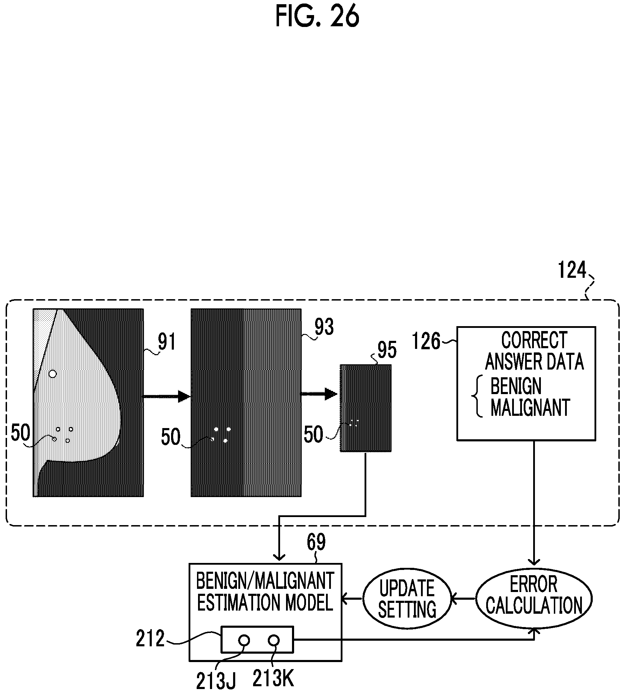

As illustrated in Fig. 26, the benign/malignant estimation model 69 is generated by training a machine learning model through machine learning using training data 124. The training data 124 is composed of a set of the low resolution image 95 and correct answer data 126. As described above, the low resolution image 95 is an image obtained by reducing the resolution of the calcification distribution image 93 representing the detection result obtained by detecting the calcification 50, from the radiation image 91 captured by the mammography apparatus 10.

The correct answer data 126 is information representing whether the calcification 50 included in the radiation image 91 is benign or malignant. **In** the present embodiment, a backpropagation method is used to perform the machine learning of the benign/malignant estimation model 69.

**In** the training of the benign/malignant estimation model 69, the low resolution image 95 in the training data 124 is input to the benign/malignant estimation model 69. It should be noted that, in the present embodiment, the training is performed by inputting the entire low resolution image 95 to the benign/malignant estimation model 69.

The benign/malignant estimation model 69 outputs an estimation result of whether the calcification 50 included in the low resolution image 95 is benign or malignant, specifically, the values of the nodes 213J and 213K included in the output layer 212 of the benign/malignant estimation model 69. In a case in which the correct answer data 126 for the low resolution image 95 input to the benign/malignant estimation model 69 is "benign", the value of the node 213J should be "1" and the value of the node 213K should be "0". In addition, in a case in which the correct answer data 126 for the low resolution image 95 input to the benign/malignant estimation model 69 is "malignant", the value of the node 213K should be "1" and the value of the node 213J should be "0".

Therefore, a difference (error) between the values of the nodes 213J and 213K output from the benign/malignant estimation model 69 and the values that should be taken by the nodes 213J and 213K corresponding to the correct answer data 126 is calculated. Then, the weight of each neuron is updated and set to reduce the error from the output layer 212 to the input layer 200 by using an error propagation method in accordance with the error, and the benign/malignant estimation model 69 is updated in accordance with the update setting.

In the training of the benign/malignant estimation model 69, the series of processing of inputting the low resolution image 95 in the training data 124 to the benign/malignant estimation model 69, outputting the respective values of the nodes 213J and 213K included in the output layer 212 from the benign/malignant estimation model 69, error calculation based on the respective values of the nodes 213J and 213K and the correct answer data 126, update setting of the weight, and updating the benign/malignant estimation model 69 is repeatedly performed.

It should be noted that the training of the benign/malignant estimation model 69 may be performed by the image processing apparatus 16, or may be performed by an external training apparatus, and the image processing apparatus 16 may acquire the trained acquired benign/malignant estimation model 69 and store the acquired benign/malignant estimation model 69 in the storage unit 62.

The low resolution image processing unit 86 according to the present modification example outputs the estimation result 113 of whether the calcification 50 is benign or malignant, which is estimated in this manner, and the determination result 110 of the type of the distribution state of the calcification to the display control unit 88.

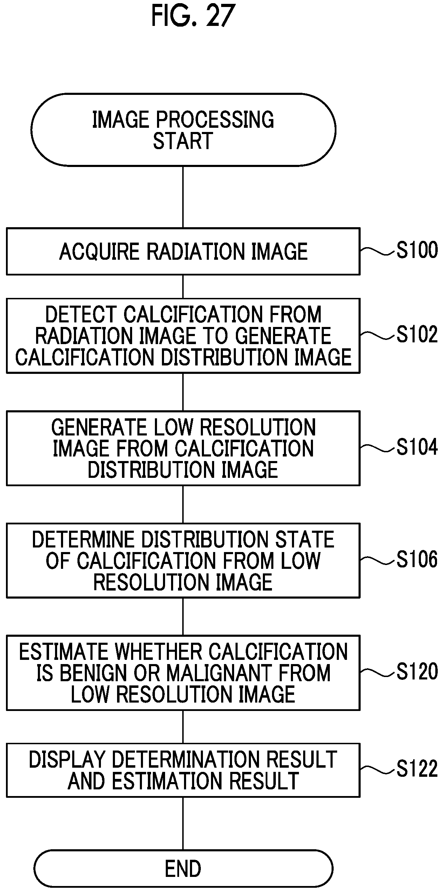

Fig. 27 illustrates a flowchart illustrating an example of a flow of image processing via the image processing apparatus 16 according to the present modification example. The image processing illustrated in Fig. 27 is different from the image processing of the above-described embodiment (see Fig. 10) in that the pieces of processing of steps S120 and S122 are provided instead of step S108.

In step S120 of Fig. 27, the low resolution image processing unit 86 estimates whether the calcification is benign or malignant from the low resolution image 94 generated in step S104. As described above, the low resolution image processing unit 86 inputs the low resolution image 94 to the benign/malignant estimation model 69, and acquires the output estimation result 113.

In next step S122, the display control unit 88 performs control of displaying, on the display unit 70, the determination result 110 of the type of the distribution state of the calcification in the step S106 and the estimation result 113 of whether the calcification is benign or malignant in the step S120. It should be noted that the display form in which the display control unit 88 displays, on the display unit 70, the type of the distribution state of the calcification and the estimation result of whether the calcification is benign or malignant is not particularly limited. In a case in which step S122 ends, the image processing illustrated in Fig. 26 ends.

In this way, according to the present modification example, it is estimated whether the calcification 50 is benign or malignant from the low resolution image 94. Therefore, the doctor or the like can comprehensively diagnose the calcification 50 with reference to the determination result of the type of the distribution state of the calcification 50 and the estimation result of whether the calcification 50 is benign or malignant.

It should be noted that, in the present modification example, the determination of the shape of the calcification 50 may be further performed. That is, the present modification example and Modification Example 7 may be combined.

As described above, in the image processing apparatus 16 according to each of the above-described embodiments, the calcification detection unit 82 detects the calcification 50 from the radiation image 90 obtained by imaging the breast by irradiating the breast with the radiation. The resolution reduction unit 84 generates the low resolution image 94 obtained by reducing the resolution to be lower than the resolution of the radiation image 90 from the calcification distribution image 92 representing the detection result of the calcification 50. The low resolution image processing unit 86 determines the type of the distribution state of the calcification 50 from the low resolution image 94.

As described above, the image processing apparatus 16 according to each of the above-described embodiments detects the calcification 50 from the radiation image 90 and reduces the resolution of the calcification distribution image 92. Therefore, even for the calcification having a weak signal, the resolution reduction can be performed without being buried in the peripheral structure. The determination of the type of the distribution state of the calcification 50 is performed from the low resolution image 94. The low resolution image 94 is an image having a smaller resolution than the radiation image 90. Therefore, the distribution can be determined from the entire low resolution image 94, and the global determination can be performed by viewing the entire image. Therefore, with the image processing apparatus 16 according to the present embodiment, it is possible to accurately determine the type of the distribution state of the calcification of the breast. In addition, according to each of the above-described embodiments, since the type in the distribution state is determined from the low resolution image 94 not including the peripheral structure, the number of the training data 120 used to train the calcification distribution determination model 64 can be reduced.

It should be noted that, in the above-described embodiment, the case has been described in which the radiation image 90 is the normal two-dimensional image, but the radiation image 90 is not limited to the normal two-dimensional image. The radiation image 90 may be a plurality of tomographic images obtained from the series of a plurality of projection images, or a composite two-dimensional image obtained by combining at least a part of the series of the plurality of projection images or the plurality of tomographic images.

It should be noted that, in the above-described embodiment, for example, as the hardware structure of the processing unit that executes various types of processing, such as the acquisition unit 80, the calcification detection unit 82, the resolution reduction unit 84, the low resolution image processing unit 86, and the display control unit 88, various processors described below can be used. As described above, in addition to the CPU that is a general-purpose processor that executes software (program) to function as various processing units, the various processors include a programmable logic device (PLD) that is a processor of which a circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of the various processors or may be configured by a combination of two or more processors of the same type or different types (for example, a combination of a plurality of FPGAs or a combination of a CPU and an FPGA). In addition, a plurality of the processing units may be configured by one processor.

A first example of the configuration in which the plurality of processing units are constituted by one processor is a form in which one processor is constituted by a combination of one or more CPUs and the software and this processor functions as the plurality of processing units, as represented by computers such as a client and a server. A second example is a form of using a processor that realizes the function of the entire system including the plurality of processing units via one integrated circuit (IC) chip, as represented by a system on a chip (SoC) or the like. In this way, as the hardware structure, the various processing units are constituted by one or more of the various processors described above.

Further, the hardware structure of these various processors is, more specifically, an electric circuit (circuitry) in which circuit elements such as semiconductor elements are combined.

In each of the above-described embodiments, the aspect has been described in which the image processing program 63 is stored (installed) in the storage unit 62 in advance, but the present disclosure is not limited thereto. The image processing program 63 may be provided in a form recorded in a recording medium such as a compact disc read-only memory (CD-ROM), a digital versatile disc read-only memory (DVD-ROM), and a universal serial bus (USB) memory. The image processing program 63 may also be downloaded from an external device via a network.

Explanation of References

1: radiography system
10: mammography apparatus
12: console

16: image processing apparatus

17: network

$19_1$ to $19_7$, 19t: irradiation position

20: radiation detector, 20A: detection surface

24: imaging table, 24A: imaging surface

26: base

27: shaft part

28: arm part

29: radiation source

30: compression plate

32: compression unit

40, 60: control unit

42, 62: storage unit

44: user I/F unit

46, 74: communication I/F unit

50, 50A, 50B, 50L, 50R: calcification, $50_1$: faint and indistinct calcification, $50_2$: small round calcification

54: tumor

56: skin line

57: nipple

60A: CPU, 60B: ROM, 60C: RAM

63: image processing program

64: calcification distribution determination model

65, 120, 121, 124: training data

66: calcification detection model

67: distribution clustering model

68: calcification shape determination model

69: benign/malignant estimation model

80: acquisition unit

82: calcification detection unit

83A: skin line detection unit, 83B: nipple detection unit

84: resolution reduction unit

86: low resolution image processing unit

88: display control unit

90, 90L, 90R, 91: radiation image

92, 92L, 92R, 93: calcification distribution image

94, 94A, 94B, 94L, 94R, 95: low resolution image

99: annotation image

110, 110A, 110B, 110L, 110R, 111: determination result

122, 123, 126: correct answer data

200: input layer

201: intermediate layer

202, 206: convolutional layer

204, 208: pooling layer

210: flat layer

211, 213A to 213K: node

212: output layer

cmp1 to cmp4: image feature map

F, F1, F2: filter

Ip: pixel of interest

DI: input data, DIc: output data

CL: normal line

U: breast

R: radiation, RC: radiation axis

$\alpha$, $\beta$: angle

**Claims**

1. An image processing apparatus (16) comprising:
   at least one processor that is configured to:

   detect calcification from a radiation image (90, 90L, 90R, 91) obtained by imaging a breast by irradiating the breast with radiation;
   generate a low resolution image (94, 94A, 94B, 94L, 94R, 95) from a calcification distribution image (92, 92L, 92R, 93) representing a detection result of the calcification by reducing a resolution of the calcification distribution image to be lower than a resolution of the radiation image (90, 90L, 90R, 91); and
   determine a type of a distribution state of the calcification based on the low resolution image (94, 94A, 94B, 94L, 94R, 95).

2. The image processing apparatus (16) according to claim 1,
   wherein the calcification distribution image (92, 92L, 92R, 93) is a grayscale image or a binary image.

3. The image processing apparatus (16) according to claim 1,
   wherein the calcification distribution image (92, 92L, 92R, 93) is a mask image in which a region other than a region of the calcification is masked.

4. The image processing apparatus (16) according to any one of claims 1 to 3,
   wherein the at least one processor is configured to enlarge an image of the calcification included in the calcification distribution image (92, 92L, 92R, 93) and generate the low resolution image (94, 94A, 94B, 94L, 94R, 95) from the calcification distribution image (92, 92L, 92R, 93) in which the image of the calcification is enlarged.

5. The image processing apparatus (16) according to any one of claims 1 to 4,
   wherein the at least one processor is configured to:

   perform filtering on the calcification distribution image (92, 92L, 92R, 93) based on a signal value of the calcification; and
   generate the low resolution image (94, 94A, 94B, 94L, 94R, 95) from the calcification distribution image (92, 92L, 92R, 93) subjected to the filtering.

6. The image processing apparatus (16) according to claim 5,
   wherein the at least one processor is configured to:

   derive a mammary gland amount of the breast from the radiation image (90, 90L, 90R, 91); and
   set a threshold value for the filtering based on the mammary gland amount.

7. The image processing apparatus (16) according to any one of claims 1 to 6,

   wherein the at least one processor is configured to detect at least one of a skin line (56) or a nipple (57) of the breast from the radiation image (90, 90L, 90R, 91), and
   the calcification distribution image (92, 92L, 92R, 93) includes at least one of the detected skin line (56) or nipple (57).

8. The image processing apparatus (16) according to any one of claims 1 to 7,
   wherein the at least one processor is configured to, in a case in which the calcification distribution image (92, 92L, 92R, 93) includes a plurality of distributions, determine the type of the distribution state for each distribution.

9. The image processing apparatus (16) according to any one of claims 1 to 8,

   wherein the radiation image (90, 90L, 90R, 91) includes a left breast radiation image (90L) obtained by imaging a left breast of a subject and a right breast radiation image (90R) obtained by imaging a right breast of the subject, and
   the at least one processor is configured to:

   detect calcification from each of the left breast radiation image (90L) and the right breast radiation image (90R);

generate a left breast low resolution image (94L) from a left breast calcification distribution image (92L) representing a detection result of the calcification of the left breast;

generate a right breast low resolution image (94R) from a right breast calcification distribution image (92R) representing a detection result of the calcification of the right breast; and

determine a type of a distribution state of the calcification based on the left breast low resolution image (94L) and the right breast low resolution image (94R).

10. The image processing apparatus (16) according to any one of claims 1 to 9,
wherein the at least one processor is configured to determine a type of a shape of the calcification based on the low resolution image (94, 94A, 94B, 94L, 94R, 95).

11. The image processing apparatus (16) according to claim 10,
wherein the at least one processor is configured to estimate a degree of malignancy of the calcification based on the type of the distribution state of the calcification and the type of the shape of the calcification.

12. The image processing apparatus (16) according to any one of claims 1 to 11,
wherein the at least one processor is configured to estimate whether the calcification is benign or malignant from the low resolution image (94, 94A, 94B, 94L, 94R, 95).

13. The image processing apparatus (16) according to any one of claims 1 to 12,
wherein the at least one processor is configured to display a determination result of the type of the distribution state in a form of being superimposed on the radiation image (90, 90L, 90R, 91).

14. The image processing apparatus (16) according to any one of claims 1 to 13,
wherein the at least one processor is configured to detect the calcification from the radiation image (90, 90L, 90R, 91) by using a rule-based calcification detection model.

15. The image processing apparatus (16) according to any one of claims 1 to 13,
wherein the at least one processor is configured to detect the calcification from the radiation image (90, 90L, 90R, 91) by using a learning-based calcification detection model.

16. The image processing apparatus (16) according to any one of claims 1 to 15,
wherein the radiation image (90, 90L, 90R, 91) is a two-dimensional image obtained by normal imaging of the breast of a subject, a plurality of tomographic images obtained from a series of a plurality of proj ection images obtained by tomosynthesis imaging of the breast, or a composite two-dimensional image obtained by combining at least a part of the series of the plurality of proj ection images or the plurality of tomographic images.

17. An image processing method performed by a computer, the method comprising:

detecting calcification from a radiation image (90, 90L, 90R, 91) obtained by imaging a breast by irradiating the breast with radiation;

generating a low resolution image (94, 94A, 94B, 94L, 94R, 95) from a calcification distribution image (92, 92L, 92R, 93) representing a detection result of the calcification by reducing a resolution of the calcification distribution image to be lower than a resolution of the radiation image (90, 90L, 90R, 91) ; and

determining a type of a distribution state of the calcification based on the low resolution image (94, 94A, 94B, 94L, 94R, 95).

18. A non-transitory storage medium storing a program that causes a computer to execute image processing, the image processing comprising:

detecting calcification from a radiation image (90, 90L, 90R, 91) obtained by imaging a breast by irradiating the breast with radiation;

generating a low resolution image (94, 94A, 94B, 94L, 94R, 95) from a calcification distribution image (92, 92L, 92R, 93) representing a detection result of the calcification by reducing a resolution of the calcification distribution image to be lower than a resolution of the radiation image (90, 90L, 90R, 91); and

determining a type of a distribution state of the calcification based on the low resolution image (94, 94A, 94B, 94L, 94R, 95).

**Patentansprüche**

1. Bildverarbeitungsvorrichtung (16), umfassend:
   mindestens einen Prozessor, der so konfiguriert ist, dass er:

   Verkalkung aus einem Strahlungsbild (90, 90L, 90R, 91), das durch Abbilden einer Brust durch Bestrahlen der Brust mit Strahlung erhalten wird, detektiert;
   ein Bild (94, 94A, 94B, 94L, 94R, 95) mit niedriger Auflösung aus einem Verkalkungsverteilungsbild (92, 92L, 92R, 93), das ein Detektionsergebnis der Verkalkung darstellt, erzeugt, indem eine Auflösung des Verkalkungsverteilungsbildes so reduziert wird, dass sie niedriger als eine Auflösung des Strahlungsbildes (90, 90L, 90R, 91) ist; und
   einen Typ eines Verteilungszustands der Verkalkung auf der Grundlage des Bildes (94, 94A, 94B, 94L, 94R, 95) mit niedriger Auflösung bestimmt.

2. Bildverarbeitungsvorrichtung (16) nach Anspruch 1,
   wobei das Verkalkungsverteilungsbild (92, 92L, 92R, 93) ein Graustufenbild oder ein Binärbild ist.

3. Bildverarbeitungsvorrichtung (16) nach Anspruch 1,
   wobei das Verkalkungsverteilungsbild (92, 92L, 92R, 93) ein Maskenbild ist, bei dem ein Bereich, der von einem Bereich der Verkalkung verschieden ist, maskiert ist.

4. Bildverarbeitungsvorrichtung (16) nach einem der Ansprüche 1 bis 3,
   wobei der mindestens eine Prozessor so konfiguriert ist, dass er ein Bild der Verkalkung, das in dem Verkalkungsverteilungsbild (92, 92L, 92R, 93) enthalten ist, vergrößert und das Bild (94, 94A, 94B, 94L, 94R, 95) mit niedriger Auflösung aus dem Verkalkungsverteilungsbild (92, 92L, 92R, 93), bei dem das Bild der Verkalkung vergrößert ist, erzeugt.

5. Bildverarbeitungsvorrichtung (16) nach einem der Ansprüche 1 bis 4,
   wobei der mindestens eine Prozessor so konfiguriert ist, dass er:

   auf dem Verkalkungsverteilungsbild (92, 92L, 92R, 93) Filterung auf der Grundlage eines Signalwerts der Verkalkung durchführt; und
   das Bild (94, 94A, 94B, 94L, 94R, 95) mit niedriger Auflösung aus dem Verkalkungsverteilungsbild (92, 92L, 92R, 93), das der Filterung unterzogen worden ist, erzeugt.

6. Bildverarbeitungsvorrichtung (16) nach Anspruch 5,
   wobei der mindestens eine Prozessor so konfiguriert ist, dass er:

   eine Menge an Brustdrüse der Brust aus dem Strahlungsbild (90, 90L, 90R, 91) ableitet; und
   einen Schwellenwert für die Filterung auf der Grundlage der Menge an Brustdrüse einstellt.

7. Bildverarbeitungsvorrichtung (16) nach einem der Ansprüche 1 bis 6,

   wobei der mindestens eine Prozessor so konfiguriert ist, dass er mindestens eine von einer Hautlinie (56) und einer Brustwarze (57) der Brust aus dem Strahlungsbild (90, 90L, 90R, 91) detektiert, und
   das Verkalkungsverteilungsbild (92, 92L, 92R, 93) mindestens eine von der detektierten Hautlinie (56) und Brustwarze (57) enthält.

8. Bildverarbeitungsvorrichtung (16) nach einem der Ansprüche 1 bis 7,
   wobei der mindestens eine Prozessor so konfiguriert ist, dass er in einem Fall, in dem das Verkalkungsverteilungsbild (92, 92L, 92R, 93) mehrere Verteilungen enthält, den Typ des Verteilungszustands für jede Verteilung bestimmt.

9. Bildverarbeitungsvorrichtung (16) nach einem der Ansprüche 1 bis 8,

   wobei das Strahlungsbild (90, 90L, 90R, 91) ein Strahlungsbild (90L) der linken Brust, das durch Abbilden einer linken Brust einer Untersuchungsperson erhalten wird, und ein Strahlungsbild (90R) der rechten Brust, das durch Abbilden einer rechten Brust der Untersuchungsperson erhalten wird, enthält, und
   der mindestens eine Prozessor so konfiguriert ist, dass er:

Verkalkung aus jeweils dem Strahlungsbild (90L) der linken Brust und dem Strahlungsbild (90R) der rechten Brust detektiert;

ein Bild (94L) mit niedriger Auflösung der linken Brust aus einem Verkalkungsverteilungsbild (92L) der linken Brust, das ein Detektionsergebnis der Verkalkung der linken Brust darstellt, erzeugt;

ein Bild (94R) mit niedriger Auflösung der rechten Brust aus einem Verkalkungsverteilungsbild (92R) der rechten Brust, das ein Detektionsergebnis der Verkalkung der rechten Brust darstellt, erzeugt; und

einen Typ eines Verteilungszustands der Verkalkung auf der Grundlage des Bildes (94L) mit niedriger Auflösung der linken Brust und des Bildes (94R) mit niedriger Auflösung der rechten Brust bestimmt.

10. Bildverarbeitungsvorrichtung (16) nach einem der Ansprüche 1 bis 9,
wobei der mindestens eine Prozessor so konfiguriert ist, dass er einen Typ einer Form der Verkalkung auf der Grundlage des Bildes (94, 94A, 94B, 94L, 94R, 95) mit niedriger Auflösung bestimmt.

11. Bildverarbeitungsvorrichtung (16) nach Anspruch 10,
wobei der mindestens eine Prozessor so konfiguriert ist, dass er einen Grad an Malignität der Verkalkung auf der Grundlage des Typs des Verteilungszustands der Verkalkung und des Typs der Form der Verkalkung schätzt.

12. Bildverarbeitungsvorrichtung (16) nach einem der Ansprüche 1 bis 11,
wobei der mindestens eine Prozessor so konfiguriert ist, dass er schätzt, ob die Verkalkung gutartig oder bösartig ist, aus dem Bild (94, 94A, 94B, 94L, 94R, 95) mit niedriger Auflösung.

13. Bildverarbeitungsvorrichtung (16) nach einem der Ansprüche 1 bis 12,
wobei der mindestens eine Prozessor so konfiguriert ist, dass er ein Bestimmungsergebnis des Typs des Verteilungszustands in einer Form anzeigt, in der es dem Strahlungsbild (90, 90L, 90R, 91) überlagert ist.

14. Bildverarbeitungsvorrichtung (16) nach einem der Ansprüche 1 bis 13,
wobei der mindestens eine Prozessor so konfiguriert ist, dass er die Verkalkung aus dem Strahlungsbild (90, 90L, 90R, 91) durch Verwenden eines regelbasierten Verkalkungsdetektionsmodells detektiert.

15. Bildverarbeitungsvorrichtung (16) nach einem der Ansprüche 1 bis 13,
wobei der mindestens eine Prozessor so konfiguriert ist, dass er die Verkalkung aus dem Strahlungsbild (90, 90L, 90R, 91) durch Verwenden eines lernbasierten Verkalkungsdetektionsmodells detektiert.

16. Bildverarbeitungsvorrichtung (16) nach einem der Ansprüche 1 bis 15,
wobei das Strahlungsbild (90, 90L, 90R, 91) ein zweidimensionales Bild ist, das durch normale Bildgebung der Brust einer Untersuchungsperson erhalten wird, mehrere Tomographiebilder, die aus einer Reihe von mehreren Projektionsbildern erhalten werden, die durch Tomosynthesebildgebung der Brust erhalten werden, oder ein zusammengesetztes zweidimensionales Bild, das durch Kombinieren mindestens eines Teils der Reihe der mehreren Projektionsbilder oder der mehreren Tomographiebilder erhalten wird.

17. Bildverarbeitungsverfahren, das von einem Computer durchgeführt wird, wobei das Verfahren umfasst:

Detektieren von Verkalkung aus einem Strahlungsbild (90, 90L, 90R, 91), das durch Bildgebung einer Brust durch Bestrahlen der Brust mit Strahlung erhalten wird;

Erzeugen eines Bildes (94, 94A, 94B, 94L, 94R, 95) mit niedriger Auflösung aus einem Verkalkungsverteilungsbild (92, 92L, 92R, 93), das ein Detektionsergebnis der Verkalkung darstellt, durch Reduzieren einer Auflösung des Verkalkungsverteilungsbildes, so dass sie niedriger als eine Auflösung des Strahlungsbildes (90, 90L, 90R, 91) ist; und

Bestimmen eines Typs eines Verteilungszustands der Verkalkung auf der Grundlage des Bildes (94, 94A, 94B, 94L, 94R, 95) mit niedriger Auflösung.

18. Nicht flüchtiges Speichermedium, das ein Programm speichert, das einen Computer veranlasst, Bildverarbeitung auszuführen, wobei die Bildverarbeitung umfasst:

Detektieren von Verkalkung aus einem Strahlungsbild (90, 90L, 90R, 91), das durch Bildgebung einer Brust durch Bestrahlen der Brust mit Strahlung erhalten wird;

Erzeugen eines Bildes (94, 94A, 94B, 94L, 94R, 95) mit niedriger Auflösung aus einem Verkalkungsverteilungsbild (92, 92L, 92R, 93), das ein Detektionsergebnis der Verkalkung darstellt, durch Reduzieren einer Auflösung des Verkalkungsverteilungsbildes, so dass sie niedriger als eine Auflösung des Strahlungsbildes (90, 90L, 90R, 91) ist; und

Bestimmen eines Typs eines Verteilungszustands der Verkalkung auf der Grundlage des Bildes (94, 94A, 94B, 94L, 94R, 95) mit niedriger Auflösung.

**Revendications**

1. Appareil de traitement d'images (16) comprenant :

   au moins un processeur qui est configuré pour :

   détecter une calcification à partir d'une image de rayonnement (90, 90L, 90R, 91) obtenue par imagerie d'un sein en irradiant le sein avec un rayonnement ;
   générer une image à basse résolution (94, 94A, 94B, 94L, 94R, 95) à partir d'une image de distribution de calcification (92, 92L, 92R, 93) représentant un résultat de détection de la calcification en réduisant une résolution de l'image de distribution de calcification pour qu'elle soit inférieure à une résolution de l'image de rayonnement (90, 90L, 90R, 91) ; et
   déterminer un type d'un état de distribution de la calcification sur la base de l'image à basse résolution (94, 94A, 94B, 94L, 94R, 95).

2. Appareil de traitement d'images (16) selon la revendication 1,
   dans lequel l'image de distribution de calcification (92, 92L, 92R, 93) est une image en niveaux de gris ou une image binaire.

3. Appareil de traitement d'images (16) selon la revendication 1,
   dans lequel l'image de distribution de calcification (92, 92L, 92R, 93) est une image de masque dans laquelle une région autre qu'une région de la calcification est masquée.

4. Appareil de traitement d'images (16) selon l'une quelconque des revendications 1 à 3,
   dans lequel l'au moins un processeur est configuré pour agrandir une image de la calcification incluse dans l'image de distribution de calcification (92, 92L, 92R, 93) et générer l'image à basse résolution (94, 94A, 94B, 94L, 94R, 95) à partir de l'image de distribution de calcification (92, 92L, 92R, 93) dans laquelle l'image de la calcification est agrandie.

5. Appareil de traitement d'images (16) selon l'une quelconque des revendications 1 à 4,
   dans lequel l'au moins un processeur est configuré pour :

   effectuer un filtrage sur l'image de distribution de calcification (92, 92L, 92R, 93) sur la base d'une valeur de signal de la calcification ; et
   générer l'image à basse résolution (94, 94A, 94B, 94L, 94R, 95) à partir de l'image de distribution de calcification (92, 92L, 92R, 93) soumise au filtrage.

6. Appareil de traitement d'images (16) selon la revendication 5,
   dans lequel l'au moins un processeur est configuré pour :

   dériver une quantité de glande mammaire du sein à partir de l'image de rayonnement (90, 90L, 90R, 91) ; et
   définir une valeur seuil pour le filtrage sur la base de la quantité de glande mammaire.

7. Appareil de traitement d'images (16) selon l'une quelconque des revendications 1 à 6,

   dans lequel l'au moins un processeur est configuré pour détecter au moins l'une d'une ligne cutanée (56) ou d'un mamelon (57) du sein à partir de l'image de rayonnement (90, 90L, 90R, 91), et
   l'image de distribution de calcification (92, 92L, 92R, 93) inclut au moins l'une de la ligne cutanée (56) détectée ou du mamelon (57) détecté.

8. Appareil de traitement d'images (16) selon l'une quelconque des revendications 1 à 7,
   dans lequel l'au moins un processeur est configuré pour, dans un cas où l'image de distribution de calcification (92, 92L, 92R, 93) inclut une pluralité de distributions, déterminer le type de l'état de distribution pour chaque distribution.

9. Appareil de traitement d'images (16) selon l'une quelconque des revendications 1 à 8,

dans lequel l'image de rayonnement (90, 90L, 90R, 91) inclut une image de rayonnement de sein gauche (90L) obtenue par imagerie d'un sein gauche d'un sujet et une image de rayonnement de sein droit (90R) obtenue par imagerie d'un sein droit du sujet, et

l'au moins un processeur est configuré pour :

détecter une calcification à partir de chacune de l'image de rayonnement de sein gauche (90L) et de l'image de rayonnement de sein droit (90R) ;

générer une image à basse résolution de sein gauche (94L) à partir d'une image de distribution de calcification de sein gauche (92L) représentant un résultat de détection de la calcification du sein gauche ;

générer une image à basse résolution de sein droit (94R) à partir d'une image de distribution de calcification de sein droit (92R) représentant un résultat de détection de la calcification du sein droit ; et

déterminer un type d'un état de distribution de la calcification sur la base de l'image à basse résolution de sein gauche (94L) et de l'image à basse résolution de sein droit (94R).

10. Appareil de traitement d'images (16) selon l'une quelconque des revendications 1 à 9,
dans lequel l'au moins un processeur est configuré pour déterminer un type d'une forme de la calcification sur la base de l'image à basse résolution (94, 94A, 94B, 94L, 94R, 95).

11. Appareil de traitement d'images (16) selon la revendication 10,
dans lequel l'au moins un processeur est configuré pour estimer un degré de malignité de la calcification sur la base du type de l'état de distribution de la calcification et du type de la forme de la calcification.

12. Appareil de traitement d'images (16) selon l'une quelconque des revendications 1 à 11,
dans lequel l'au moins un processeur est configuré pour estimer si la calcification est bénigne ou maligne à partir de l'image à basse résolution (94, 94A, 94B, 94L, 94R, 95).

13. Appareil de traitement d'images (16) selon l'une quelconque des revendications 1 à 12,
dans lequel l'au moins un processeur est configuré pour afficher un résultat de détermination du type de l'état de distribution sous une forme superposée sur l'image de rayonnement (90, 90L, 90R, 91).

14. Appareil de traitement d'images (16) selon l'une quelconque des revendications 1 à 13,
dans lequel l'au moins un processeur est configuré pour détecter la calcification à partir de l'image de rayonnement (90, 90L, 90R, 91) en utilisant un modèle de détection de calcification basé sur des règles.

15. Appareil de traitement d'images (16) selon l'une quelconque des revendications 1 à 13,
dans lequel l'au moins un processeur est configuré pour détecter la calcification à partir de l'image de rayonnement (90, 90L, 90R, 91) en utilisant un modèle de détection de calcification basé sur l'apprentissage.

16. Appareil de traitement d'images (16) selon l'une quelconque des revendications 1 à 15,
dans lequel l'image de rayonnement (90, 90L, 90R, 91) est une image bidimensionnelle obtenue par imagerie normale du sein d'un sujet, une pluralité d'images tomographiques obtenues à partir d'une série d'une pluralité d'images de projection obtenues par imagerie par tomosynthèse du sein, ou une image bidimensionnelle composite obtenue en combinant au moins une partie de la série de la pluralité d'images de projection ou de la pluralité d'images tomographiques.

17. Procédé de traitement d'images effectué par un ordinateur, le procédé comprenant :

détecter une calcification à partir d'une image de rayonnement (90, 90L, 90R, 91) obtenue par imagerie d'un sein en irradiant le sein avec un rayonnement ;

générer une image à basse résolution (94, 94A, 94B, 94L, 94R, 95) à partir d'une image de distribution de calcification (92, 92L, 92R, 93) représentant un résultat de détection de la calcification en réduisant une résolution de l'image de distribution de calcification pour qu'elle soit inférieure à une résolution de l'image de rayonnement (90, 90L, 90R, 91) ; et

déterminer un type d'un état de distribution de la calcification sur la base de l'image à basse résolution (94, 94A, 94B, 94L, 94R, 95).

**18.** Support de stockage non transitoire stockant un programme qui amène un ordinateur à exécuter un traitement d'images, le traitement d'images comprenant :

détecter une calcification à partir d'une image de rayonnement (90, 90L, 90R, 91) obtenue par imagerie d'un sein en irradiant le sein avec un rayonnement ;

générer une image à basse résolution (94, 94A, 94B, 94L, 94R, 95) à partir d'une image de distribution de calcification (92, 92L, 92R, 93) représentant un résultat de détection de la calcification en réduisant une résolution de l'image de distribution de calcification pour qu'elle soit inférieure à une résolution de l'image de rayonnement (90, 90L, 90R, 91) ; et

déterminer un type d'un état de distribution de la calcification sur la base de l'image à basse résolution (94, 94A, 94B, 94L, 94R, 95).

FIG. 1

## FIG. 2

## FIG. 3

## FIG. 4

| DISTRIBUTION / MORPHOLOGY | DIFFUSE /SCATTERED | REGIONAL | CLUSTERED | LINEAR | SEGMENTAL |
|---|---|---|---|---|---|
| SMALL ROUND | CATEGORY 2 | | CATEGORY 3 OR 4 | | |
| FAINT AND INDISTINCT | CATEGORY 2 | | CATEGORY 3 OR 4 | | |
| POLYMORPHOUS /NON-UNIFORM | CATEGORY 3 | | CATEGORY 4 | CATEGORY 5 | |
| FINE LINEAR /FINE BRANCHING | CATEGORY 5 | | | | |

# FIG. 5

EP 4 454 566 B1

## FIG. 6

## FIG. 7

# FIG. 8

DETERMINATION RESULT
110
(DISTRIBUTION STATE)

DIFFUSE/SCATTERED
REGIONAL
CLUSTERED
LINEAR
SEGMENTAL

64 CALCIFICATION DISTRIBUTION DETERMINATION MODEL

86 LOW RESOLUTION IMAGE PROCESSING UNIT

94

50

84 RESOLUTION REDUCTION UNIT

92

50

82 CALCIFICATION DETECTION UNIT

90

54

50

# FIG. 9

16

| 80 | 82 | 84 | 86 | 88 |
|---|---|---|---|---|
| ACQUISITION UNIT | CALCIFICATION DETECTION UNIT | RESOLUTION REDUCTION UNIT | LOW RESOLUTION IMAGE PROCESSING UNIT | DISPLAY CONTROL UNIT |

IMAGE PROCESSING APPARATUS

# FIG. 10

IMAGE PROCESSING START

ACQUIRE RADIATION IMAGE — S100

DETECT CALCIFICATION FROM RADIATION IMAGE TO GENERATE CALCIFICATION DISTRIBUTION IMAGE — S102

GENERATE LOW RESOLUTION IMAGE FROM CALCIFICATION DISTRIBUTION IMAGE — S104

DETERMINE DISTRIBUTION STATE OF CALCIFICATION FROM LOW RESOLUTION IMAGE — S106

DISPLAY DETERMINATION RESULT — S108

END

## FIG. 11A

## FIG. 11B

# FIG. 12

~62

### STORAGE UNIT

IMAGE PROCESSING PROGRAM — 63

CALCIFICATION DISTRIBUTION DETERMINATION MODEL — 64

CALCIFICATION DETECTION MODEL — 66

# FIG. 13

# FIG. 14

CALCIFICATION ENLARGEMENT PROCESSING

EP 4 454 566 B1

# FIG. 15

```
        ╭──────────────────────────╮
        │     IMAGE PROCESSING     │
        │          START           │
        ╰──────────────────────────╯
                     │
   ┌─────────────────────────────────────┐
   │      ACQUIRE RADIATION IMAGE        │────S100
   └─────────────────────────────────────┘
                     │
   ┌─────────────────────────────────────┐
   │     DETECT CALCIFICATION FROM       │
   │   RADIATION IMAGE TO GENERATE       │────S102
   │ CALCIFICATION DISTRIBUTION IMAGE    │
   └─────────────────────────────────────┘
                     │
   ┌─────────────────────────────────────┐
   │   ENLARGE IMAGE OF CALCIFICATION    │
   │      INCLUDED IN CALCIFICATION      │────S103
   │        DISTRIBUTION IMAGE           │
   └─────────────────────────────────────┘
                     │
   ┌─────────────────────────────────────┐
   │       GENERATE LOW RESOLUTION       │
   │    IMAGE FROM CALCIFICATION         │────S104
   │        DISTRIBUTION IMAGE           │
   └─────────────────────────────────────┘
                     │
   ┌─────────────────────────────────────┐
   │  DETERMINE DISTRIBUTION STATE       │
   │   OF CALCIFICATION FROM LOW         │────S106
   │        RESOLUTION IMAGE             │
   └─────────────────────────────────────┘
                     │
   ┌─────────────────────────────────────┐
   │     DISPLAY DETERMINATION RESULT    │────S108
   └─────────────────────────────────────┘
                     │
        ╭──────────────────────────╮
        │           END            │
        ╰──────────────────────────╯
```

# FIG. 16

CALCIFICATION DETECTION UNIT `82`

FILTERING PROCESSING

RESOLUTION REDUCTION UNIT `84`

LOW RESOLUTION IMAGE PROCESSING UNIT `86`

CALCIFICATION DISTRIBUTION DETERMINATION MODEL `64`

DETERMINATION RESULT (DISTRIBUTION STATE) `110`

DIFFUSE/SCATTERED
REGIONAL
CLUSTERED
LINEAR
SEGMENTAL

EP 4 454 566 B1

# FIG. 17

```
          ┌─────────────────────┐
          │   IMAGE PROCESSING  │
          │        START        │
          └─────────────────────┘
                     │
          ┌─────────────────────┐
          │ ACQUIRE RADIATION   │ ─── S100
          │       IMAGE         │
          └─────────────────────┘
                     │
          ┌─────────────────────┐
          │ DETECT CALCIFICATION│
          │ FROM RADIATION IMAGE│ ─── S102
          │ TO GENERATE         │
          │ CALCIFICATION       │
          │ DISTRIBUTION IMAGE  │
          └─────────────────────┘
                     │
          ┌─────────────────────┐
          │ PERFORM FILTERING   │
          │ PROCESSING ON       │ ─── S103A
          │ CALCIFICATION       │
          │ DISTRIBUTION IMAGE  │
          └─────────────────────┘
                     │
          ┌─────────────────────┐
          │ GENERATE LOW        │
          │ RESOLUTION IMAGE    │ ─── S104
          │ FROM CALCIFICATION  │
          │ DISTRIBUTION IMAGE  │
          └─────────────────────┘
                     │
          ┌─────────────────────┐
          │ DETERMINE           │
          │ DISTRIBUTION STATE  │
          │ OF CALCIFICATION    │ ─── S106
          │ FROM LOW RESOLUTION │
          │ IMAGE               │
          └─────────────────────┘
                     │
          ┌─────────────────────┐
          │ DISPLAY DETERMINATION│ ─── S108
          │ RESULT              │
          └─────────────────────┘
                     │
          ┌─────────────────────┐
          │         END         │
          └─────────────────────┘
```

# FIG. 18

EP 4 454 566 B1

## FIG. 19

EP 4 454 566 B1

# FIG. 20

# FIG. 21

**90R**

54

50R

**82**
CALCIFICATION
DETECTION
UNIT

**92R**

50R

**84**
RESOLUTION
REDUCTION
UNIT

**94R**

50R

**86**
LOW RESOLUTION
IMAGE
PROCESSING UNIT

**64**
CALCIFICATION
DISTRIBUTION
DETERMINATION
MODEL

**110R**
DETERMINATION RESULT
(DISTRIBUTION STATE)

DIFFUSE/SCATTERED
REGIONAL
CLUSTERED
LINEAR
SEGMENTAL

**90L**

50L

54

**82**
CALCIFICATION
DETECTION
UNIT

**92L**

50L

**84**
RESOLUTION
REDUCTION
UNIT

50L

**94L**

**110L**
DETERMINATION RESULT
(DISTRIBUTION STATE)

DIFFUSE/SCATTERED
REGIONAL
CLUSTERED
LINEAR
SEGMENTAL

EP 4 454 566 B1

# FIG. 22

CALCIFICATION DETECTION UNIT 82

RESOLUTION REDUCTION UNIT 84

LOW RESOLUTION IMAGE PROCESSING UNIT 86

CALCIFICATION SHAPE DETERMINATION MODEL 68

DETERMINATION RESULT (SHAPE) 111

SMALL ROUND
FAINT AND INDISTINCT
POLYMORPHOUS
/NON-UNIFORM
FINE LINEAR
/FINE BRANCHING

# FIG. 23

# FIG. 24

```
┌─────────────────────────┐
│   IMAGE PROCESSING      │
│        START            │
└─────────────────────────┘
             │
┌─────────────────────────┐
│ ACQUIRE RADIATION IMAGE │ ──── S100
└─────────────────────────┘
             │
┌─────────────────────────┐
│ DETECT CALCIFICATION FROM│
│ RADIATION IMAGE TO GENERATE│ ──── S102
│ CALCIFICATION DISTRIBUTION IMAGE│
└─────────────────────────┘
             │
┌─────────────────────────┐
│  GENERATE LOW RESOLUTION │
│  IMAGE FROM CALCIFICATION│ ──── S104
│    DISTRIBUTION IMAGE    │
└─────────────────────────┘
             │
┌─────────────────────────┐
│ DETERMINE DISTRIBUTION STATE│
│  OF CALCIFICATION FROM LOW│ ──── S106
│     RESOLUTION IMAGE     │
└─────────────────────────┘
             │
┌─────────────────────────┐
│   DETERMINE SHAPE OF     │
│  CALCIFICATION FROM LOW  │ ──── S110
│     RESOLUTION IMAGE     │
└─────────────────────────┘
             │
┌─────────────────────────┐
│ ESTIMATE CATEGORY FROM TYPE│
│  OF DISTRIBUTION STATE OF│
│ CALCIFICATION AND TYPE OF│ ──── S112
│     SHAPE THEREOF        │
└─────────────────────────┘
             │
┌─────────────────────────┐
│ DISPLAY DETERMINATION RESULT│ ──── S114
│      AND CATEGORY        │
└─────────────────────────┘
             │
┌─────────────────────────┐
│          END            │
└─────────────────────────┘
```

# FIG. 25

# FIG. 26

# FIG. 27

```
        ┌─────────────────────────┐
        │    IMAGE PROCESSING     │
        │        START            │
        └─────────────────────────┘
                    │
        ┌─────────────────────────┐
        │ ACQUIRE RADIATION IMAGE │────S100
        └─────────────────────────┘
                    │
        ┌─────────────────────────┐
        │   DETECT CALCIFICATION  │
        │   FROM RADIATION IMAGE  │────S102
        │   TO GENERATE           │
        │ CALCIFICATION           │
        │ DISTRIBUTION IMAGE      │
        └─────────────────────────┘
                    │
        ┌─────────────────────────┐
        │  GENERATE LOW RESOLUTION│
        │  IMAGE FROM             │────S104
        │  CALCIFICATION          │
        │  DISTRIBUTION IMAGE     │
        └─────────────────────────┘
                    │
        ┌─────────────────────────┐
        │ DETERMINE DISTRIBUTION  │
        │ STATE OF CALCIFICATION  │────S106
        │ FROM LOW RESOLUTION     │
        │ IMAGE                   │
        └─────────────────────────┘
                    │
        ┌─────────────────────────┐
        │ ESTIMATE WHETHER        │
        │ CALCIFICATION IS BENIGN │────S120
        │ OR MALIGNANT FROM       │
        │ LOW RESOLUTION IMAGE    │
        └─────────────────────────┘
                    │
        ┌─────────────────────────┐
        │ DISPLAY DETERMINATION   │
        │ RESULT AND ESTIMATION   │────S122
        │ RESULT                  │
        └─────────────────────────┘
                    │
        ┌─────────────────────────┐
        │          END            │
        └─────────────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018161405 A
- JP 2016022143 A

- JP 2008086389 A
- JP 2010051456 A

**Non-patent literature cited in the description**

- Multi-task Graph Convolutional Neural Network for Calcification Morphology and Distribution Analysis in Mammograms. **HAO, DU et al.** arxiv.org. Cornell University Library, 14 May 2021